# EUROPEAN PATENT SPECIFICATION

(11) **EP 3 652 538 B1**
(45) Date of publication and mention of the grant of the patent: **06.03.2024**
(21) Application number: 18831634.3
(22) Date of filing: 11.07.2018
(51) Int. Cl.: G01N 33/50, C12Q 1/68

(54) **SMALL RNA PREDICTORS FOR HUNTINGTON'S DISEASE**
KLEINE RNA-PRÄDIKTOREN FÜR MORBUS HUNTINGTON
PETITS PRÉDICTEURS D'ARN POUR LA MALADIE DE HUNTINGTON

(30) Priority: 11.07.2017 US 201762530968 P
(43) Date of publication of application: 20.05.2020
(73) Proprietor: Gatehouse Bio, Inc., Natick, MA 01760 (US)
(72) Inventor: SALZMAN, David, Needham, MA 02494 (US)
(74) Representative: Grund, Martin
(86) International application number: PCT/US2018/041687
(87) International publication number: WO 2019/014375

(56) References cited:
- WO-A1-2012/005572
- WO-A1-2018/136936
- US-A1- 2015 197 809
- US-A1- 2017 145 511
- US-A1- 2017 145 511
- MÓNICA BAÑEZ-CORONEL ET AL: "A Pathogenic Mechanism in Huntington's Disease Involves Small CAG-Repeated RNAs with Neurotoxic Activity", PLOS GENETICS, vol. 8, no. 2, 23 February 2012 (2012-02-23), page e1002481, XP055463256, DOI: 10.1371/journal.pgen.1002481
- DATABASE Nucleotide 26 June 2006 (2006-06-26), "Bos taurus microRNA bta-miR-10b, complete sequence", XP055566499, retrieved from NCBI Database accession no. DQ642619.1
- DATABASE Nucleotide 26 March 2016 (2016-03-26), "Tor tambroides miR-10b-5p miRNA, complete sequence", XP055566500, retrieved from NCBI Database accession no. KT921539.1

## Description

### BACKGROUND

Huntington's disease (HD) is an inherited disorder affecting the viability of brain cells. The earliest symptoms often begin between the ages of 30 and 50, and typically include changes in cognition and behavior, and subsequently a lack of coordination. As the disease advances, uncoordinated body movements become more apparent. Physical abilities gradually worsen, and mental abilities generally decline into dementia. While most individuals with Huntington's disease eventually exhibit similar physical symptoms, the onset, progression and extent of cognitive and behavioral symptoms vary significantly between individuals.

HD is caused by an autosomal dominant mutation in either of an individual's two copies of the Huntingtin gene. Part of the gene is a repeated section called a trinucleotide repeat (CAG), which varies in length between individuals and may change length between generations. When the length of this repeated section reaches a certain threshold, it produces an altered form of the protein having a longer polyglutamine tract (or polyQ tract), called mutant Huntingtin protein (mHTT). Pathological changes are attributed to the mHTT protein. Most people have fewer than 36 repeated glutamines in the polyQ region which results in production of the cytoplasmic protein Huntingtin. However, a sequence of 36 or more glutamines results in the production of a protein which has different characteristics. Mutant Huntingtin is expressed throughout the body and is associated with abnormalities in peripheral tissues, including muscle atrophy, cardiac failure, impaired glucose tolerance, weight loss, osteoporosis, and testicular atrophy.

mHTT increases the decay rate of certain types of neurons. Regions of the brain have differing amounts and reliance on these types of neurons, and are affected accordingly. Generally, the number of CAG repeats is related to how much this process is affected, and accounts for about 60% of the variation of the age of the onset of symptoms. The remaining variation is attributed to environment and other genes that modify the mechanism of HD.

Medical diagnosis of the onset of HD can be made following the appearance of physical symptoms specific to the disease. Genetic testing can confirm a physical diagnosis if there is no family history of HD. Even before the onset of symptoms, genetic testing can confirm if an individual carries an expanded copy of the trinucleotide repeat in the HTT gene that causes the disease. The genetic test for HD consists of a blood test which counts the numbers of CAG repeats in each of the HTT alleles. Forty or more CAG repeats is considered a full penetrance allele (FPA), and is considered a "positive test." A positive result is not considered a diagnosis, since it may be decades before the symptoms begin. However, a negative test means that the individual does not carry the expanded copy of the gene and will not develop HD. A person who tests positive for the disease will likely develop HD sometime within their lifetime. A trinucleotide repeat length of 36 to 39 repeats is considered an incomplete or reduced penetrance allele (RPA). It may cause symptoms, usually later in the adult life. There is a risk of about 60% that a person with an RPA will be symptomatic at the age of 65 years, and a 70% risk of being symptomatic at the age of 75 years. A trinucleotide repeat length of 27 to 35 repeats is considered an intermediate allele (IA), or large normal allele. It is not associated with symptomatic disease in the tested individual, but may expand upon further inheritance to give symptoms in offspring. 26 or fewer repeats is not associated with HD.

There is no cure for HD, but there are treatments available that may reduce the severity of some symptoms. There is some evidence for the usefulness of physical therapy, occupational therapy, and speech therapy. Tetrabenazine was approved in 2008 for treatment of chorea in Huntington's disease in the US. Other drugs that help to reduce chorea include neuroleptics and benzodiazepines. Compounds such as amantadine or remacemide are still under investigation and have shown preliminary positive results. Hypokinesia and rigidity, especially in juvenile cases, can be treated with antiparkinsonian drugs, and myoclonic hyperkinesia can be treated with valproic acid. Psychiatric symptoms can be treated with medications similar to those used in the general population. Selective serotonin reuptake inhibitors and mirtazapine have been recommended for depression, while atypical antipsychotic drugs are recommended for psychosis and behavioral problems. Specialist neuropsychiatric input is recommended as people may require long-term treatment with multiple medications in combination. While these therapies treat symptoms of HD, none of them are designed to target or correct the underlying genetics of the disease, or specifically alter the biology of mHTT.

Thus, the identification of disease-modifying therapies is the main objective for pharmaceutical intervention and drug discovery. However, these efforts are hampered by the fact that there are no clinically meaningful biomarkers to aid in drug discovery and development. Such biomarkers need to be accessible, prognostic, and/or disease-specific. Discovery and investigation of therapeutic interventions, including pharmaceutical interventions, would benefit from the availability of biomarkers correlative of underlying disease processes.

Diagnostic tests to evaluate Huntington's disease activity are needed, for example, to aid treatment and decision making in affected individuals, as well as for use as biomarkers in drug discovery and clinical trials, including for patient enrollment, stratification, and disease monitoring.

US 2015/197809 A1 discloses methods for the disanosis, prognosis, and treatment of neurological conditions, e.g. Huntington's Disease and Parkinson's disease, relating to the misregulation of miRNAs in such conditions.

Banez-Cornel et al., 2012, PLOS GENETICS, discloses a possible link between Huntiington's Disease and small CAG-repeated RNA expression with an aberrant activation of siRNA/miRNA gene silencing machinery.

US 2017/145511A1 discloses discloses methods for the disanosis, prognosis, and treatment of neurological conditions, e.g. Huntington's Disease, relating to the misregulation of miRNAs in such conditions.

WO 2012/005572 A1 discloses diagnostic and therapeutic uses of a miRNA molecule, in a disease and condition associated with melanoma or a disease or a condition associated with activated BRAF pathway.

WO 2018/136936 A1 discloses a method for identifying or detecting small RNA predictors of a disease or condition.

### SUMMARY OF THE INVENTION

The invention is defined by the appended claims.

The present disclosure provides methods and kits for evaluating Huntington's disease (HD) activity, including in patients undergoing treatment for HD or a candidate treatment for HD, as well as in animal and cell models. Specifically, the present disclosure provides biomarkers (sRNA predictors) that are binary predictors of disease activity, and are useful for detecting and/or evaluating HD disease stage, grade, progression, prognosis, and response to therapy or candidate therapy. The biomarkers are further useful in the context of drug discovery and clinical trials, to identify candidate pharmaceutical interventions (or other therapies) that are useful for the treatment or management of disease (e.g., treatment or progression monitoring).

The invention involves detecting binary small RNA (sRNA) predictors of Huntington's disease or Huntington's disease activity according to SEQ ID NO: 6 or SEQ ID NO:
1, in cells or in a biological sample from a subject or patient. The sRNA sequences are identified as being present in samples of an HD experimental cohort, while not being present in any samples of a comparator cohort ("positive sRNA predictors"). The invention thereby detects sRNAs that are binary predictors, exhibiting 100% Specificity for Huntington's disease activity.

In one aspect, the invention provides a method for evaluating HD in a subject. The method comprises providing a biological sample from a subject having an expanded trinucleotide repeat in a Huntingtin gene, or providing RNA extracted therefrom, and determining the presence or absence of the sRNA predictors according to SEQ ID NO: 6 or SEQ ID NO: 1 in the sample. The presence of the sRNA predictors is indicative of Huntington's disease activity.

The positive sRNA predictors may further include one or more sRNA predictors from Tables 2, 3, 4, and 5 (SEQ ID NOS: 1-137). For example, the positive sRNA predictors may further include one or more sRNA predictors from Table 2 (SEQ ID NOS: 1 to 29), which were identified in HD patients, but were absent from healthy controls, and Parkinson's disease controls. In some embodiments, the relative or absolute amount of the one or more predictors is correlative with disease stage or severity. In some embodiments, the positive sRNA predictors further include one or more sRNA predictors from Table 3 (SEQ ID NOS: 30 to 102), which were identified in patients with a specified grade of HD. In some embodiments, the number of predictors that is present in a sample, or the accumulation of one or more of the predictors, directly correlates with the progression of HD or underlying severity of disease or active symptoms. In some embodiments, the positive sRNA predictors further include one or more sRNA predictors from Tables 4A and/or 4B (SEQ ID NOS: 1, 2, 7, 11-13, 15, 18, 19, 20, 24, 48, 53-55, 61, 103-136), which discriminate fast and slow progressing disease, respectively. In some embodiments, the positive sRNA predictors further include one or more from Table 5 (SEQ ID NO:1 2, 3, 6, 7, 9, 10, 11, 12, 22, 23, 25, 26, 27, 55, 40, 41, and 137), which were further validated in fluid samples.

In some embodiments, the presence or absence of at least 1, 2, 3, 4, or 5 sRNAs, or at least 10 sRNAs, or at least 40 sRNAs from one or more of Table 2, Table 3, Table 4, or Table 5 are determined (SEQ ID NOS: 1-137). In some embodiments, the presence or absence of at least one negative sRNA predictor is also determined, which are identified in non-HD samples, such as healthy controls. In some embodiments, a panel of sRNAs comprising positive predictors from Table 2 or Table 5 is tested against the sample. In some embodiments, the panel may comprise at least 2, or at least 5, or at least 10, or at least 20, or at least 25 sRNAs from Table 2. In some embodiments, the panel comprises all sRNAs from Table 2 or Table 5. For example, a sample may be positive for at least about 2, 3, 4, or 5 sRNA predictors in Table 2, indicating active disease, with more severe or advanced disease being correlative with about 10, 15 or about 20 sRNA predictors. In some embodiments, the relative or absolute amount of the sRNA predictors in Table 2 or Table 5 are directly correlative with disease grade or severity.

Generally, the presence of at least 1, 2, 3, 4, or 5 positive predictors with the absence of all the negative predictors is predictive of HD activity. In some embodiments, a panel of 5 to about 100, or about 5 to about 60, sRNA predictors are tested against the sample. In these embodiments, the panel may optionally comprise assays for at least 5 (e.g., about 8) positive sRNA predictors. While not each experimental sample will be positive for each positive predictor, the panel is large enough to provide at least 90% or nearly 100% coverage for the condition in an HD cohort.

sRNA predictors can be identified or detected in any biological samples, including solid tissues and/or biological fluids. sRNA predictors can be identified or detected in animals (e.g., vertebrates and invertebrates), or in some embodiments, cultured cells or the media of cultured cells. For example, the sample may be a biological fluid sample from a human or animal subject (e.g., a mammalian subject), such as blood, serum, plasma, urine, saliva, or cerebrospinal fluid. In some embodiments, the sample is a solid tissue such as brain tissue.

In various embodiments, detection of the sRNA predictors involves one of various detection platforms, which can employ reverse-transcription, amplification, and/or hybridization of a probe, including quantitative or qualitative PCR, or Real-Time PCR. PCR detection formats can employ stem-loop primers for RT-PCR in some embodiments, and optionally in connection with fluorescently-labeled probes. In some embodiments, sRNAs are detected by a hybridization assay or RNA sequencing.

The invention involves detection of sRNA predictors in cells or animals (or samples derived therefrom) that contain a mutant Huntingtin gene. In various embodiments, the number and/or identity of the sRNA predictors, or the relative amount thereof, is correlative with disease activity for patients, subjects, or cells having a full penetrance HTT allele, or a reduced penetrance HTT allele, or an intermediate penetrance allele. In some embodiments, the sRNA predictor is indicative of HD biological processes in patients or subjects that are otherwise considered Asymptomatic.

In some aspects, the invention provides the use of for evaluating samples for Huntington's disease activity. The kits comprise sRNA-specific probes and/or primers configured for detecting sRNAs according to SEQ ID NO: 6 or SEQ ID NO: 1. In some embodiments, the kit comprises sRNA-specific probes and/or primers configured for detecting at least 5, or at least 10, or at least 20, or at least 40 sRNAs listed in Tables 2, 3, 4, and/or 5 (SEQ ID NOS: 1-137).

Other aspects and embodiments of the invention will be apparent from the following detailed description.

### DESCRIPTION OF THE FIGURES

FIG. 1 shows the read count of disease-specific biomarkers per HD grade for the sRNA predictors listed in Table 2, demonstrating greater accumulation of sRNA predictor biomarkers as HD progresses.
FIG. 2 depicts an unsupervised hierarchical clustering plot of the top 200 highest frequency small RNAs after cross-validation from GSE64977 in the Experimental (Huntington's Disease, N = 28) and Comparator (non-Huntington's Disease, N = 36) group. Samples were plotted on the x-axis and small RNAs were plotted on the y-axis.
FIG. 3 shows validation of binary, small RNA predictors of Huntington's Disease. 18 Small RNAs from 32 healthy controls and 32 mixed Grade Huntington's Disease brains were analyzed by RT-qPCR (Applied Biosystems). 1000ng of total RNA was used for multiplex-RT, and 1/2500^{th} was used to test each small RNA in triplicate by qPCR (max Ct = 50.000000). All samples had a Ct of <39.999999, and had a minimum of 2 of 3 replicates with a coefficient of variance <5%.
FIG. 4 depicts validation of disease monitoring small RNA biomarkers in the frontal cortex. Ct values for each small RNA biomarker were binned according to Vonstattel Grade. HDB-4, HDB-5, and HDB-7 showed statistical significance by Analysis of Variance (ANOVA, p≤0.05) using 4-degrees of freedom.
FIG. 5 shows validation of small RNA biomarkers in CSF. Small RNA biomarkers were tested in CSF from 15 Controls, 10 Pre-Low, 10-Pre-Med, 10 Pre-High, and 15 Huntington's Disease patients by RT-qPCR.
FIG. 6 depicts validation of disease monitoring small RNA biomarkers in CSF. Ct values for each small RNA biomarker were binned according to CAP_{D} Group. HDB-1 and HDB-17 showed statistical significance by Analysis of Variance (ANOVA, p=0.001) using 4-degrees of freedom.

### DESCRIPTION OF THE TABLES

Tables 1A to 1C characterize patient cohorts, including Huntington's disease (HD) cohort (Table 1A), Healthy control cohort (Table 1B), and a control Parkinson's disease (PD) cohort (Table 1C).

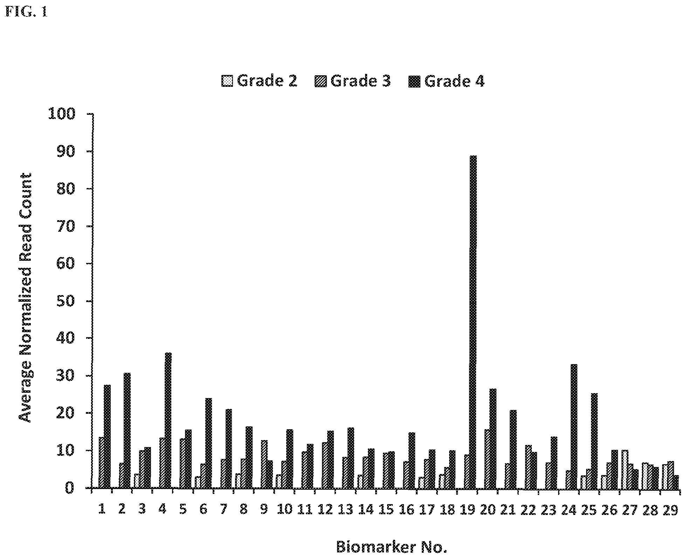

Tables 2 shows 29 sRNA positive predictors for HD (Experimental Group is HD Grade 2, 3, and 4; Comparator Group is PRE-HD, Healthy, and PD). Table 2A shows positive predictors for HD regardless of Grade. Table 2B shows the average read count of the 29 positive predictors in each disease grade (2, 3, and 4). These results are further illustrated in Figure 1.

Tables 3 shows discovery of positive sRNA predictors by HD Grade. Where the Experimental Group is Asymptomatic CAG-repeat carriers (PRE-HD); the Comparator Group is HD Grade 2, 3, or 4, Healthy, or PD. Where the Experimental Group is HD Grade 2; Comparator Group is HD Grade 3 or 4, PRE-HD, Healthy, and PD. Where the Experimental Group is HD Grade 3; the Comparator Group is HD Grade 2 or 4, PRE-HD, Healthy, and PD. Where the Experimental Group is HD Grade 4; the Comparator Group is HD Grade 2, 3, PRE-HD, Healthy, and PD.

Table 4 shows HD prognostic biomarkers. Table 4A shows <10 year prognosis biomarkers (fast progression biomarkers). Table 4B shows >20 year prognosis biomarkers (slow progression biomarkers).

Table 5 shows a panel of 18 biomarkers validated in independent samples, including fluid samples.

Table 6 depicts primers and probes used for RT-qPCR analysis of binary small RNA classifiers. All sequences are 5' to 3'. Lowercase letters in the RT primer indicate the 6-nucleotide sequence that anneals to the target small RNA to initiate reverse transcription. TaqMan probes contain a 5' 6-Carboxyfluorescein (6FAM) fluorescent dye, and a 3' non-fluorescent quencher (NFQ) covalently linked to a minor groove binder (MGB). HDB = Huntington's Disease Biomarker.

### DETAILED DESCRIPTION OF THE INVENTION

The invention is defined by the appended claims.

The present disclosure provides methods and kits for evaluating Huntington's disease (HD) in a subject. In an embodiment, the subject is undergoing treatment for HD or a candidate treatment for HD, as well as in animal and cell models. Specifically, the present disclosure provides biomarkers (sRNA predictors) that are binary predictors of disease activity, and are useful for detecting and/or evaluating underlying disease processes, disease grade, progression, and response to therapy or candidate therapy. The biomarkers are further useful in the context of drug discovery and clinical trials, to identify candidate therapies that are useful for treatment of HD or HD symptoms, as well as to select or stratify patients, and monitor disease progression.

In one aspect, the invention involves detecting binary small RNA (sRNA) predictors of Huntington's disease or Huntington's disease activity having SEQ ID NO: 6 or SEQ ID NO: 1, in a biological sample. The sRNA sequences are identified as being present in samples of an HD experimental cohort, while not being present in any samples in a comparator cohort. These sRNA markers are termed "positive sRNA predictors", and by definition provide 100% Specificity. In some embodiments, the method further comprises detecting one or more sRNA sequences that are present in one or more samples of the comparator cohort, and which are not present in any of the samples of the experimental cohort. These predictors are termed "negative sRNA predictors", and provide additional level of confidence to the predictions. In contrast to detecting dysregulated sRNAs (such as miRNAs that are up- or down-regulated), the invention identifies sRNAs that are binary predictors for Huntington's disease activity.

small RNA species ("sRNAs") are non-coding RNAs less than 200 nucleotides in length, and include microRNAs (miRNAs) (including iso-miRs), Piwi-interacting RNAs (piRNAs), small interfering RNAs (siRNAs), vault RNAs (vtRNAs), small nucleolar RNAs (snoRNAs), transfer RNA-derived small RNAs (tsRNAs), ribosomal RNA-derived small RNA fragments (rsRNAs), small rRNA-derived RNAs (srRNA), and small nuclear RNAs (U-RNAs), as well as novel uncharacterized RNA species. Generally, "iso-miR" refers to those sequences that have variations with respect to a reference miRNA sequence (e.g., as used by miRBase). In miRBase, each miRNA is associated with a miRNA precursor and with one or two mature miRNA (-5p and -3p). Deep sequencing has detected a large amount of variability in miRNA biogenesis, meaning that from the same miRNA precursor many different sequences can be generated. There are four main variations of iso-miRs: (1) 5' trimming, where the 5' cleavage site is upstream or downstream from the referenced miRNA sequence; (2) 3' trimming, where the 3' cleavage site is upstream or downstream from the reference miRNA sequence; (3) 3' nucleotide addition, where nucleotides are added to the 3' end of the reference miRNA; and (4) nucleotide substitution, where nucleotides are changed from the miRNA precursor.

U.S. Provisional Patent Application No. 62/449,275 filed on January 23, 2017, and PCT/US2018/014856 filed January 23, 2018, disclose processes for identifying sRNA predictors. The process includes computational trimming of 3' adapters from RNA sequencing data, and sorting data according to unique sequence reads.

In one aspect, the invention provides a method for evaluating Huntington's disease (HD) in a subject. The method comprises providing a biological sample from a subject or patient having an expanded trinucleotide repeat in a Huntingtin gene, or providing RNA extracted therefrom, and determining the presence or absence of sRNA predictors having SEQ ID NO: 6 or SEQ ID NO: 1 in the sample. The presence of the sRNA predictors is indicative of Huntington's disease activity.

The term "Huntington's disease activity" refers to active disease processes that result (directly or indirectly) in HD symptoms and overall decline in cognition, behavior, and/or motor skills and coordination. The term Huntington's disease activity can further refer to the relative health of affected cells, and particularly cells expressing the mutant HTT protein. In some embodiments, the HD activity is indicative of neuron viability.

In an embodiment, the positive sRNA predictors further include one or more sRNA predictors from Tables 2, 3, 4, or 5 (SEQ ID NOS: 1-137). Sequences disclosed herein are shown as the reverse transcribed DNA sequence. For example, the positive sRNA predictors may further include one or more sRNA predictors from Table 2 (SEQ ID NOS: 1-29), which are indicative of HD and/or HD stage. In some embodiments, the positive sRNA predictors further include one or more sRNA predictors from Table 3 (SEQ ID NOS: 30 to 102), which are indicative of HD stage (as shown in Table 3). In some embodiments, the positive sRNA predictors further include one or more from Table 4 (SEQ ID NOS: 1, 2, 7, 11-13, 15, 18, 19, 20, 24, 48, 53-55, 61, 103-136), which are indicative of fast progressing (Table 4A) or slow progressing (Table 4B) disease. In some embodiments, the sRNA predictors comprise one or more from Table 5 (SEQ ID NO: 1, 2, 3, 6, 7, 9, 10, 11, 12, 22, 23, 25, 26, 27, 55, 40, 41, and 137).

Specifically, Tables 2A and 2B show 29 sRNA positive predictors for HD. These 29 sRNA predictors were present in a cohort of HD Grade 2, 3, and 4 samples (as the Experimental Group), but were not present in any of the Comparator Group samples, which were comprised of Asymptomatic patients (PRE-HD), Healthy, and Parkinson's Disease (PD) samples. Table 2A shows positive predictors for HD regardless of grade. The 29 positive predictors were each present in from 23% to 50% of HD samples, and by definition, each positive predictor provides 100% Specificity for the presence of HD in the cohort. 18 of the 29 positive predictors for HD are iso-miRs of miR-10b. 3 of the 29 positive predictors are iso-miRs of miR-196a-2. Table 2B shows the average read count across HD grade for the 29 predictors (shown graphically in Figure 1). In some embodiments, the number of predictors that is present in a sample directly correlates with the progression of HD.

Tables 3 show discovery of positive sRNA predictors by HD Grade. For example, Table 3 lists positive sRNA predictors identified: (1) in Asymptomatic CAG-repeat carriers (PRE-HD) as the Experimental Group, with the Comparator Group including samples from HD Grade 2, 3, or 4, healthy individuals (Healthy), or Parkinson's Disease (PD); (2) HD Grade 2 samples as the Experimental Group, with the Comparator Group being HD Grade 3 or 4, PRE-HD, Healthy, and PD;. (3) HD Grade 3 samples as the Experimental Group, where the Comparator Group is HD Grade 2 or 4, PRE-HD, Healthy, and PD; and (4) Experimental Group with HD Grade 4, where the Comparator Group is HD Grade 2, 3, PRE-HD, Healthy, and PD.

In various embodiments, the presence or absence of at least five sRNAs are determined, including positive and negative predictors and other potential controls. In some embodiments, the presence or absence of at least 8 sRNAs, or at least 10 sRNAs, or at least about 50 sRNAs are determined. The total number of sRNAs determined, in some embodiments, is less than about 1000 or less than about 500, or less than about 200, or less than about 100, or less than about 50. Therefore, the presence or absence of sRNAs can be determined using any number of specific molecular detection assays.

In some embodiments, the presence or absence of at least 2, or at least 5, or at least 10 sRNAs from Table 2, Table 3, Table 4, and/or Table 5 are determined (SEQ ID NOS: 1-137). In some embodiments, the presence or absence of at least one negative sRNA predictor is also determined. In some embodiments, a panel of sRNAs comprising positive predictors from Table 2 are determined, and the panel may comprise at least 2, at least 5, at least 10, or at least 20 sRNAs from Table 2. In some embodiments, the presence or absence of one or more iso-miRs of miR-10b is determined. In some embodiments, the panel comprises all sRNAs from Table 2. In some embodiments, a panel of sRNAs comprising positive predictors from Table 3 are determined, and the panel may comprise at least 2, at least 5, at least 10, or at least 20 sRNAs from Table 3, which are specific for HD grade. In some embodiments, the panel comprises all sRNAs from Table 3. In some embodiments, a panel of sRNAs comprising positive predictors from Table 4 are determined, and the panel may comprise at least 2, at least 5, at least 10, or at least 20 sRNAs from Table 4, which are specific for fast-progressing (Table 4A) and slow-progressing (Table 4B) disease. In some embodiments, the panel comprises all sRNAs from Table 4. In some embodiments, the panel of biomarkers comprises at least 1, 2, or 5 sRNAs from Table 5.

In some embodiments, the one or more (or all) positive sRNA predictors are present in at least about 10% of HD samples, or at least about 20% of HD samples, or at least about 30% of HD samples, or at least about 40% of HD samples. In some embodiments, the identity and/or number of predictors identified correlates with active disease processes. For example, a sample may be positive for at least 1, 2, 3, 4, or 5 sRNA predictors in Table 2, indicating active disease, with more severe or advanced disease processes being correlative with about 10, or at least about 15, or at least about 20 sRNA predictors in Table 2. In some embodiments, the absolute level (e.g., sequencing read count) or relative level (e.g., using a qualitative assay such as Real Time PCR) is determined for the sRNA predictors in Table 2 or Table 5, which can be correlative with disease grade.

In some embodiments, samples that test negative for the presence of the positive sRNA predictors, test positive for at least 1, or at least about 5, or at least about 10, or at least about 20, or at least about 30, or at least about 40, or at least about 50, or at least about 100 negative sRNA predictors. Negative predictors can be specific for healthy individuals or other disease states (such as PD or dementia). Individuals testing positive for HD, will typically not test positive for the presence of any negative predictors.

Generally, the presence of at least 1, 2, 3, 4, or 5 positive predictors, and the absence of all of the negative predictors is predictive of HD activity. In some embodiments, a panel of from 5 to about 100, or from about 5 to about 60 sRNA predictors are detected in the sample. While not each experimental sample will be positive for each positive predictor, the panel is large enough to provide at least about 75%, at least about 80%, at least about 85%, at least about 90%, at least about 95%, or about 100% coverage for the condition in an HD cohort.

In various embodiments, detection of the sRNA predictors involves one of various detection platforms, which can employ reverse-transcription, amplification, and/or hybridization of a probe, including quantitative or qualitative PCR, or RealTime PCR. PCR detection formats can employ stem-loop primers for RT-PCR in some embodiments, and optionally in connection with fluorescently-labeled probes. In some embodiments, sRNAs are detected by RNA sequencing, with computational trimming of the 3' sequencing adaptor.

Generally, a real-time polymerase chain reaction (qPCR) monitors the amplification of a targeted DNA molecule during the PCR, i.e. in real-time. Real-time PCR can be used quantitatively, and semi-quantitatively. Two common methods for the detection of PCR products in real-time PCR are: (1) non-specific fluorescent dyes that intercalate with any double-stranded DNA (e.g., SYBR Green (I or II), or ethidium bromide), and (2) sequence-specific DNA probes consisting of oligonucleotides that are labelled with a fluorescent reporter which permits detection only after hybridization of the probe with its complementary sequence (e.g. TAQMAN).

In some embodiments, the assay format is TAQMAN real-time PCR. TAQMAN probes are hydrolysis probes that are designed to increase the Specificity of quantitative PCR. The TAQMAN probe principle relies on the 5' to 3' exonuclease activity of Taq polymerase to cleave a dual-labeled probe during hybridization to the complementary target sequence, with fluorophore-based detection. TAQMAN probes are dual labeled with a fluorophore and a quencher, and when the fluorophore is cleaved from the oligonucleotide probe by the Taq exonuclease activity, the fluorophore signal is detected (e.g., the signal is no longer quenched by the proximity of the labels). As in other quantitative PCR methods, the resulting fluorescence signal permits quantitative measurements of the accumulation of the product during the exponential stages of the PCR. The TAQMAN probe format provides high Sensitivity and Specificity of the detection.

In some embodiments, sRNA predictors present in the sample are converted to cDNA using specific primers, e.g., a stem-loop primer. Amplification of the cDNA may then be quantified in real time, for example, by detecting the signal from a fluorescent reporting molecule, where the signal intensity correlates with the level of DNA at each amplification cycle.

Alternatively, sRNA predictors in the panel, or their amplicons, are detected by hybridization. Exemplary platforms include surface plasmon resonance (SPR) and microarray technology. Detection platforms can use microfluidics in some embodiments, for convenient sample processing and sRNA detection.

Generally, any method for determining the presence of sRNAs in samples can be employed. Such methods further include nucleic acid sequence based amplification (NASBA), flap endonuclease-based assays, as well as direct RNA capture with branched DNA (QuantiGene^{™}), Hybrid Capture^{™} (Digene), or nCounter^{™} miRNA detection (nanostring). The assay format, in addition to determining the presence of miRNAs and other sRNAs may also provide for the control of, inter alia, intrinsic signal intensity variation. Such controls may include, for example, controls for background signal intensity and/or sample processing, and/or hybridization efficiency, as well as other desirable controls for detecting sRNAs in patient samples (e.g., collectively referred to as "normalization controls").

In some embodiments, the assay format is a flap endonuclease-based format, such as the Invader^{™} assay (Third Wave Technologies). In the case of using the invader method, an invader probe containing a sequence specific to the region 3' to a target site, and a primary probe containing a sequence specific to the region 5' to the target site of a template and an unrelated flap sequence, are prepared. Cleavase is then allowed to act in the presence of these probes, the target molecule, as well as a FRET probe containing a sequence complementary to the flap sequence and an auto-complementary sequence that is labeled with both a fluorescent dye and a quencher. When the primary probe hybridizes with the template, the 3' end of the invader probe penetrates the target site, and this structure is cleaved by the Cleavase resulting in dissociation of the flap. The flap binds to the FRET probe and the fluorescent dye portion is cleaved by the Cleavase resulting in emission of fluorescence.

In some embodiments, RNA is extracted from the sample prior to sRNA processing for detection. RNA may be purified using a variety of standard procedures as described, for example, in RNA Methodologies, A laboratory guide for isolation and characterization, 2nd edition, 1998, Robert E. Farrell, Jr., Ed., Academic Press. In addition, there are various processes as well as products commercially available for isolation of small molecular weight RNAs, including mirVANA^{™} Paris miRNA Isolation Kit (Ambion), miRNeasy^{™} kits (Qiagen), MagMAX^{™} kits (Life Technologies), and Pure Link^{™} kits (Life Technologies). For example, small molecular weight RNAs may be isolated by organic extraction followed by purification on a glass fiber filter. Alternative methods for isolating miRNAs include hybridization to magnetic beads. Alternatively, miRNA processing for detection (e.g., cDNA synthesis) may be conducted in the biofluid sample, that is, without an RNA extraction step.

In some embodiments, the presence or absence of the sRNAs are determined by nucleic acid sequencing, and individual sRNAs are identified by a process that comprises computational trimming a 3' sequencing adaptor from individual sRNA sequences. See U. S. Provisional Patent Application No. 62/449,275 filed on January 23, 2017, and PCT/US2018/014856 filed January 23, 2018.

Generally, assays can be constructed such that each assay is at least 98% specific for the sRNA (e.g., iso-miR) over an annotated sequence and/or other non-predictive iso-miRs and sRNAs. Annotated sequences can be determined with reference to miRBase. For example, in preparing sRNA predictor-specific real-time PCR assays, PCR primers and fluorescent probes can be prepared and tested for their level of Specificity. Bicyclic nucleotides or other modifications involving the 2' position (e.g., LNA, cET, and MOE), or other nucleotide modifications (including base modifications) can be employed in probes to increase the Sensitivity or Specificity of detection.

sRNA predictors can be identified in any biological samples, including solid tissues and/or biological fluids. sRNA predictors can be identified in animals (e.g., vertebrate and invertebrate subjects), or in some embodiments, cultured cells or media from cultured cells. For example, the sample is a biological fluid sample from human or animal subjects (e.g., a mammalian subject), such as blood, serum, plasma, urine, saliva, or cerebrospinal fluid. miRNAs can be found in biological fluid, as a result of a secretory mechanism that may play an important role in cell-to-cell signaling. See, Kosaka N, et al., Circulating microRNA in body fluid: a new potential biomarker for cancer diagnosis and prognosis, Cancer Sci. 2010; 101: 2087-2092). miRs from cerebrospinal fluid and serum have been profiled according to conventional methods with the goal of stratifying patients for disease status and pathology features. Burgos K, et al., Profiles of Extracellular miRNA in Cerebrospinal Fluid and Serum from Patients with Alzheimer's and Parkinson's Diseases Correlate with Disease Status and Features of Pathology, PLOS ONE Vol. 9, Issue 5 (2014). In some embodiments, the sample is a solid tissue sample, which may comprise neurons. In some embodiments, the tissue sample is a brain tissue sample, such as from the frontal cortex region. In some embodiments, sRNA predictors are identified in at least two different types of samples, including brain tissue and a biological fluid such as blood.

In an embodiment, the method involves detection of sRNA predictors in cells or animals that exhibit a Huntington's disease genotype or phenotype. In various embodiments, the number and/or identity of the sRNA predictors is correlative with disease activity for patients or subjects having a full penetrance HTT allele, or a reduced penetrance HTT allele, or an intermediate penetrance allele. In some embodiments, the sRNA predictor is indicative of HD biological processes in patients or subjects that are otherwise considered Asymptomatic.

In some embodiments, the sRNA predictors are indicative of Grade 1 HD disease processes. Grade 1 HD is usually from 0 to 8 years from illness onset. In Grade 1, HD patients maintain only marginal engagement in occupation, and maintain typical pre-disease level of independence in all other basic functions, such as financial management, domestic responsibilities, and activities of daily living (eating, dressing, bathing, etc.); or perform satisfactorily in typical salaried employment and requires slight assistance in only one basic function: finances, domestic chores, or activities of daily living.

In some embodiments, the sRNA predictors are indicative of Grade 2 HD disease processes. Grade 2 HD is often from 3 to 13 years from illness onset. Subjects with Grade 2 HD, are typically unable to work but require only slight assistance in all basic functions: finances, domestic, daily activities, or unable to work and requiring major assistance in one basic function with only slight assistance needed in one other basic function; one basic function is handled independently.

In some embodiments, the sRNA predictors are indicative of Stage 3 HD disease processes. Grade 3 HD is typically from 5 to 16 years from illness onset. Individuals with Grade 3 HD are totally unable to engage in employment and require major assistance in most basic functions: financial affairs, domestic responsibilities, and activities of daily living.

In some embodiments, the sRNA predictors are indicative of Grade 4 HD disease processes. Grade 4 HD is typically from 9 to 21 years from illness onset. Individuals with Grade 4 HD require major assistance in financial affairs, domestic responsibilities, and most activities of daily living. For instance, comprehension of the nature and purpose of procedures may be intact, but major assistance is required to act on them. Care may be provided at home but needs may be better provided for at an extended care facility.

In some embodiments, the method is repeated to determine the sRNA predictor profile over time, for example, to determine the impact of a therapeutic regimen, or a candidate therapeutic regimen. For example, a subject or patient may be evaluated at a frequency of at least about once per year, or at least about once every six months, or at least once per month, or at least once per week. In some embodiments, a decline in the number of predictors present over time, or a slower increase in the number of predictors detected over time, is indicative of slower disease progression or milder disease symptoms. Embodiments of the invention are useful for constructing animal models for HD treatment, as well as useful as biomarkers in human clinical trials.

In another aspect, the invention provides kits for evaluating samples for Huntington's disease activity, comprising sRNA-specifc probes and/or primers configured for performing the method disclosed herein, for detecting sRNAs having SEQ ID NO: 6 or SEQ ID NO: 1. In various embodiments, the kits further comprise sRNA-specific probes and/or primers configured for detecting a plurality of sRNAs listed in Tables 2, 3, 4, and/or 5 (SEQ ID NOS: 1-137). In some embodiments, the kit comprises sRNA-specific probes and/or primers configured for detecting at least 2, at least 5, or at least 10, or at least 20, or at least 40 sRNAs listed in Tables 2, 3, 4, and/or 5 (SEQ ID NOS: 1-137). In some embodiments, the kit comprises sRNA-specific probes and/or primers configured for detecting at least 2, 3, 4, 5, or at least 10, or at least 20 sRNAs listed in Table 2 (SEQ ID NOS: 1-29). In some embodiments, the kit comprises sRNA-specific probes and/or primers configured for detecting at least 2, 3, 4, 5, or at least 10, or at least 20, or at least 40 sRNAs listed in Table 3 (SEQ ID NOS: 30-102). In some embodiments, the kit comprises sRNA-specific probes and/or primers configured for detecting at least 2, 3, 4, 5, or at least 10, or at least 20 sRNAs listed in Table 4 (SEQ ID NOS: 1, 2, 7, 11-13, 15, 18, 19, 20, 24, 48, 53-55, 61, 103-136). In some embodiments, the kit comprises sRNA-specific probes and/or primers configured for detecting at least 1, 2, 3, 4, 5, or more (incuding all) sRNAs from Table 5 (SEQ ID NO: 1, 2, 3, 6, 7, 9, 10, 11, 12, 22, 23, 25, 26, 27, 55, 40, 41, and 137).

The kits may comprise probes and/or primers suitable for a quantitative or qualitative PCR assay, that is, for specific sRNA predictors. In some embodiments, the kits comprise a fluorescent dye or fluorescent-labeled probe, which may optionally comprise a quencher moiety. In some embodiments, the kit comprises a stem-loop RT primer. In some embodiments, the kit may comprise an array of sRNA-specific hybridization probes.

In some embodiments, the invention provides a kit comprising reagents for detecting a panel of from 5 to about 100 sRNA predictors, or from about 5 to about 50 sRNA predictors, or from 5 to about 20 sRNAs. In these embodiments, the kit may comprise at least 5, at least 10, at least 20 sRNA predictor assays (e.g., reagents for such assays). In various embodiments, the kit comprises at least 10 positive predictors and at least 5 negative predictors. In some embodiments, the kit comprises a panel of at least 5, or at least 10, or at least 20, or at least 40 sRNA predictor assays, the sRNA predictors being selected from Table 2, Table 3, Table 4, and/or Table 5. In some embodiments, at least 1 sRNA predictor is selected from Table 2 or Table 5. In some embodiments, at least one sRNA predictor is an iso-miR of miR-10b. Such assays may comprise reverse transcription (RT) primers, amplification primers and probes (such as fluorescent probes or dual labeled probes) specific for the sRNA predictors over annotated sequences as well as other (non-predictive) variations. In some embodiments, the kit is in the form of an array or other substrate containing probes for detection of sRNA predictors by hybridization.

Other aspects and embodiments of the invention will be apparent from the following examples.

### EXAMPLES

### Example 1: Binary classifiers for Huntington's Disease were identified in either an Experimental or Comparator Group

To identify binary small RNA predictors for Huntington's Disease, small RNA sequencing data from the GEO Database Accession Number GSE64977 and GSE72962 were downloaded.

GSE64977 contains small RNA sequencing data derived from the frontal cortex region BA9 from: 26 post-mortem verified and disease graded Huntington's Disease patients:

| Number of Samples | Post-Mortem Verified Disease/Grade | GEO Database Accession Number |
|---|---|---|
| 2 | Asymptomatic CAG-Repeat Expansion Carriers (PRE-HD) | GSE64977 |
| 4 | Grade 2 | GSE64977 |
| 15 | Grade 3 | GSE64977 |
| 7 | Grade 4 | GSE64977 |

from: Hoss AG, Labadorf A, Latourelle JC, Kartha VK et al. miR-10b-5p expression in Huntington's disease brain relates to age of onset and the extent of striatal involvement. BMC Med Genomics 2015 Mar 1;8:10.

GSE72962 contains small RNA sequencing data derived from the frontal cortex region BA9 from: 36 Healthy Control Donors and 29 Parkinson's Disease Patients:

| Number of Samples | Sample Type | GEO Database Accession Number |
|---|---|---|
| 36 | Healthy Control Donors (Healthy) | GSE72962 |
| 29 | Parkinson's Disease (PD) | GSE72962 |

from: Hoss AG, Labadorf A, Beach TG, Latourelle JC et al. microRNA Profiles in Parkinson's Disease Prefrontal Cortex. Front Aging Neurosci 2016;8:36.

Files were converted from a .sra to .fastq format using the SRA Tool Kit v2.8.0 for Mac, and .fastq formatted files were processed using a software platform developed by sRNAlytics, LLC as described in U.S. Patent Application No. 15/877,989 and International Application No. PCT/US2018/014856, filed on January 23, 2018. Specifically, all .fastq data files were processed by trimming adapter sequences using the (Regex) regular expression-based search and trim algorithm, where 5' TGGAATTCTCGGGTGCCAAGGAA 3' (containing up to a 15 nucleotide 3'-end truncation) was input as the 3' adapter sequence. Parameters for Regex searching permitted up to (i) 4 wild-cards, (ii) 1 insertion, (iii) 2 deletions, (iv) 1 deletion and 1 wild-card, (v) 1 insertion and 1 wild-card. Specific biomarker panels containing binary small RNA predictors (present in samples of the Experimental Group, but not present in any samples of the Comparator Group) were identified as follows:

### (1) HD vs non-HD (Tables 2A to 2D)

Experimental Group = HD Grade 2, 3, and 4 (N = 26)
Comparator Group = Healthy, and PD (N = 65)

### (2) HD Grade Stratification (Tables 3A to 3D)

(A) Experimental Group = PRE-HD (N = 2)
   Comparator Group = HD Grade 2, 3, 4, Healthy, and PD (N = 91)
(B) Experimental Group = HD Grade 2 (N = 4)
   Comparator Group = HD Grade 3, 4, PRE-HD, Healthy, and PD (N = 87)
(C) Experimental Group = HD Grade 3 (N = 15)
   Comparator Group = HD Grade 2, 4, PRE-HD, Healthy, and PD (N = 76)
(D) Experimental Group = HD Grade 4 (N = 7)
   Comparator Group = HD Grade 2, 3, PRE-HD, Healthy, and PD (N = 86)

### (3) Fast vs Slow Progressors

(A) Fast Progression (patients who lived <10years)
   Experimental Group = HD Patients who lived <10 years (N = 5)
   Comparator Group = HD Patients who lived >20 years, Healthy, and
   PD (N = 88)
(B) Slow Progression (patients who lived >20years)
   Experimental Group = HD Patients who lived >20years (N = 16)
   Comparator Group = HD Patients who lived <10years, Healthy, and
   PD (N = 72)

Binary small RNA predictors identified in the Experimental Group all have 100% Specificity, by definition. Therefore, panels of binary small RNA predictors were selected according to the following criteria to give the best Specificity when considered as individual/independent biomarkers: (1) frequency of occurrence in the Experimental Group; (2) highest read count; (3) the nature of the small RNA (preference was given to small RNAs that mapped back to the human genome using either the Basic Local Alignment Tool (BLAT) or Basic Local Alignment Search Tool (BLAST) algorithms); each sample (i.e. patient) in the Experimental Group had to have a minimum of 5 binary small RNA predictors.

Probability scores (p-values) were calculated for each individual binary small RNA predictor using a Chi-Square 2x2 Contingency Table and one-tailed Fisher's Exact Probability Test.

Probability scores (p-values) were calculated for panels of binary small RNA predictor for each Experimental Group using a Chi-Square 2x2 Contingency Table and one-tailed Fisher's Exact Probability Test (all giving 100% Specificity and 100% Sensitivity).

FIG. 1 shows the read count of disease-specific biomarkers per HD grade for the sRNA predictors listed in Table 2, demonstrating greater accumulation of sRNA predictor biomarkers as HD progresses.

### Example 2: Validation of Binary Small RNA Predictors

Binary small RNA classifiers were identified using the method described in Example 1.

Binary small RNA classifiers from the Experimental Group were cross-validated against an additional 890 non-Huntington's Disease fluid samples. The Cross-Validation Set contains sequencing data from healthy controls, as well as other non-Huntington neurodegenerative diseases (i.e. Alzheimer's and Parkinson's) as disease mimics, with samples depicted below:

| **Post-Mortem Verified Disease/Stage** | | **Biofluid** | **Samples (N)** | | **Accession Number** | **Ref** |
|---|---|---|---|---|---|---|
| **Healthy Control Donors (CTL)** | | - | **587** | | - | - |
| | *Control* | CSF | ***68*** | | phs000727 | 7 |
| | *Control* | Serum | ***204*** | | - | - |
| | | | | *70* | phs000727 | 7 |
| | | | | *38* | GSE100467 | 8 |
| | | | | *96* | GSE113994 | 9 |
| | *Control* | Plasma | ***216*** | | GSE113994 | 9 |
| | *Control* | PAXgene | ***99*** | | - | - |
| | | | | *22* | GSE46579 | 10 |
| | | | | *77* | GSE100467 | 8 |
| **Non-Huntington's Disease Neurological Disorders** | | - | **303** | | - | - |
| | | - | **177** | | - | - |
| | *Alzheimer's Disease* | CSF | | *67* | phs000727 | 7 |
| | | Serum | | *62* | phs000727 | 7 |
| | | PAXgene | | *48* | GSE46579 | 10 |
| | *Parkinson's Disease* | - | **33** | | - | - |
| | | CSF | | *17* | phs000727 | 7 |
| | | Serum | | *16* | phs000727 | 7 |
| | *Parkinson's Disease with Dementia* | - | **46** | | - | - |
| | | CSF | | *24* | phs000727 | 7 |
| | | Serum | | *22* | phs000727 | 7 |
| | *Parkinson's Disease with Alzheimer's Disease* | - | **47** | | - | - |
| | | CSF | | *25* | phs000727 | 7 |
| | | Serum | | *22* | phs000727 | 7 |

The binary small RNA classifiers that remained after cross-validation were then mapped to the miRbase pre-microRNA reference library (See, Kozomara A, Griffiths-Jones S. miRBase: annotating high confidence microRNAs using deep sequencing data. *NAR* 2014 43:D68-D73. PMID: 24275495) (i.e. the template) using the following criteria: a perfect match within 4 nucleotides of the 5'-end and 11 nucleotides of the 3'-end of an annotated gene, allowing up to 2 internal mismatches (where the mismatched nucleotides were restricted to either A>G or C>T), and allowing up to 2 non-templated additions to the 5'-end and up to 4 non-templated additions to the 3'-end were considered mapped to an annotated gene.

Mapped reads were scaled to Reads Per Million Mapped Reads (RPM) by dividing the read count of each small RNA by the total number of mapped reads, then multiplying by 1 million to get the number of reads expected if there were exactly 1 million read depth.

Quantile normalization was performed on RPM values to improve cross-sample consistency and relative comparability. To do this, the mean value of the highest marker across all samples was calculated and each marker:: sample pair was assigned an expression value and normalized to that. This process was repeated on the next-highest RPM until all the mapped reads were normalized. Ties were resolved by averaging normalized values across multiple reads. Zero-read values were left as zero.

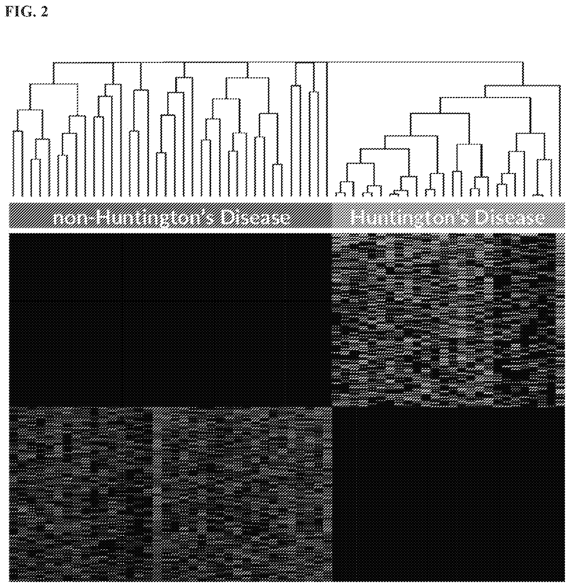

The top 200 binary small RNA classifiers for Huntington's and non-Huntington's Disease were then analyzed by unsupervised, hierarchical clustering, as depicted in FIG. 2. Binary small RNA classifiers were selected from the Experimental Group according to the following criteria to give the best Sensitivity when considered as an independent or paneled biomarker test: (1) frequency of occurrence in the Experimental Group; (2) read count; (3) preference to small RNAs mapping to the miRBase reference (as described above), such as (a) small RNAs with non-templated 3' uridines (U) were weighted highest, (b) small RNAs with non-templated 3' adenosines (A) were weighted the second highest, and (c) small RNAs with non-templated 5' nucleotides were weighted third highest; and (4) positive or negative correlation with Vonstattel Disease Grade.

Probability scores (p-values) were calculated for each individual binary small RNA predictor using a Chi-Square 2x2 Contingency Table and one-tailed Fisher's Exact Probability Test.

### Example 3: RT-qPCR Validation of Binary Small RNA Predictors

### Primary Validation in Frontal Cortex Brodmann Area

Frozen brain tissue from prefontal cortex Brodmann Area 9 (BA9) was obtained from Dr. Richard H. Myers of the Boston University Medical School. 32 neurologically-normal control samples, and 32 Huntington's Disease samples were selected for primary validation. All subjects had no evidence of Alzheimer's or Parkinson's Disease comorbidity based on neuropathology reports. For microscopic examination, 16 tissue blocks were systematically taken and histologically assessed as previously described (See, Vonstattel JP, Myers RH, Stevens TJ, Ferrante RJ, et al. Neuropathological classification of Huntington's Disease. J Neuropathol Exp Neurol. 1985 Nov;44(6):559-77. PMID: 2932539). HD samples and controls were not different from postmortem interval PMI (average 18.1 ±6.75) and or death age (60.8 ±13.45). CAG repeat size, Age at Onset, Age at Death was unknown for all but 8 of the Huntington's Disease samples.

Total RNA was isolated using QIAzol Lysis Reagent and purified using miRNeasy MinElute Cleanup columns (Qiagen Sciences Inc). RNA Integrity Number (RIN) and RNA quantity for RT-qPCR was assessed using an Agilent BioAnalyzer 2100 and RNA 6000 Nano Kit. RIN is calculated by measuring the area under the peak for 18S and 28S RNA as a ratio of total RNA, as well as the relative height of the 18S and 28S peaks to determine RNA quality. The RIN/RQN values were similar for all 64 samples (RIN = 7.6 ±0.75).

Table 6 shows the primers and probes used for RT-qPCR analysis of 18 binary small RNA classifiers. Each Custom TaqMan small RNA Assay comes with 2 tubes: (1) a target-specific hairpin RT primer (e.g., 5x concentration), and (2) a premixed set of target-specific forward and reverse PCR primers, and TaqMan probe (e.g., 20x concentration). The TaqMan probe has a 6-carboxyfluorescein (6FAM) fluorescent dye at the 5'-end, and a non-fluorescent quencher (NFQ) covalently linked to a minor groove binder (MGB) on the 3'-end.

Reverse Transcription reactions were carried out in in multiplex by pooling RT primers to a final concentration of 0.1x, according to the manufacturer's protocol. Total RNA (0.1ug) of each sample was reverse transcribed using the RT primer pool and the TaqMan MicroRNA Reverse Transcription Kit (Applied Biosystems) in a 15uL reaction, according to the manufacturer's protocol. Following incubation, RT reactions were diluted 1:250 with 10mM Tris pH 8.0. 2uL of each RT reaction were analyzed by qPCR in a 10uL reaction volume in a 384-well fast microplate using TaqMan Universal Master Mix II, no UNG (Applied Biosystems). Reactions were cycled in an ABI 7900 HT Fast Real-Time PCR machine using a max Cycle Threshold (Ct-value) of 50.000000. All samples were analyzed in triplicate.

Samples had to pass the following acceptance criteria before being included in the final statistical analysis:
1. Ct values had to be <39.999999
2. each sample had to have a minimum of 2 replicates
3. the coefficient of variance (%CV) between sample replicates had to be <5%

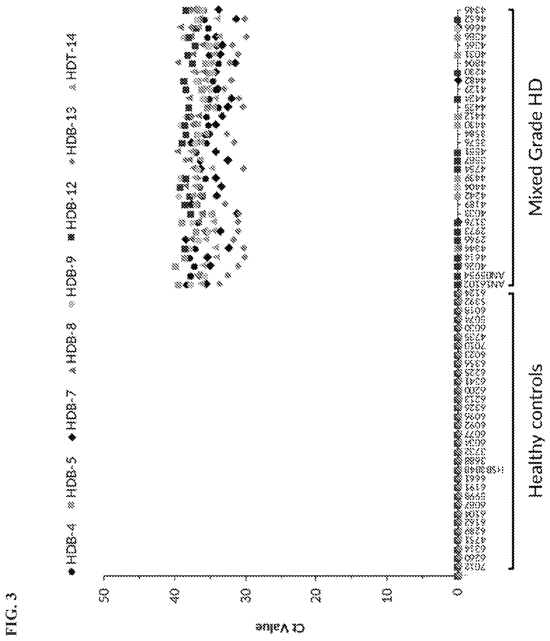

Results showed that 8 of 18 small RNAs were completely binary in that they only scored in the Huntington's Disease samples, but not the Control sample, as depicted in FIG. 3. Statistical analysis of each of the 8 binary small RNA predictors showed that there was 100% Specificity and 100% Sensitivity with a panel of 8 small RNAs (p=1.09×10⁻¹⁸). This panel gave a minimum of 2x coverage per Huntington's Disease sample across the validation set.

Statistical analysis was performed done using a Chi-Square and two-tailed Fisher's Exact Probability Test. Since small RNAs were not detected in the healthy control samples (i.e. 100% Specificity) we were unable to calculate Odds Ratios. HDB = Huntington's Disease Biomarker:

| **Biomarker** | **Specificity** | **Sensitivity** | **p-value** |
|---|---|---|---|
| HDB-4 | 100% | 100% | 1.09×10⁻¹⁸ |
| HDB-5 | 100% | 59% | 7.97×10⁻⁸ |
| HDB-7 | 100% | 91% | 7.14×10⁻¹⁵ |
| HDB-8 | 100% | 88% | 6.43×10⁻¹⁴ |
| HDB-9 | 100% | 81% | 3.01×10⁻¹² |
| HDB-12 | 100% | 59% | 7.97×10⁻⁸ |
| HDB-13 | 100% | 100% | 1.09×10⁻¹⁸ |
| HDB-14 | 100% | 97% | 3.60×10⁻¹⁷ |
| Panel of 8 | 100% | 100% | 1.09×10⁻¹⁸ |

Of the 8 binary small RNA classifiers, 5 classifiers had a Pearson Correlation >0.8500. Mean Ct values for each biomarker were calculated per Vonstattel Grade. Values were plotted for each Vonstattel Grade and Linear Regression Coefficients (R²) were calculated for each biomarker. Statistical significance of the variance between Vonstattel Grade was determined by ANOVA test with 4 degrees of freedom. N.S. = not significant:

| **Biomarker** | **Mean Pre-HD** | **Mean Grade 2** | **Mean Grade 3** | **Mean Grade 4** | **R²** | **ANOVA** |
|---|---|---|---|---|---|---|
| **Biomarker4** | 38.0493615 | 36.88814677 | 35.57429543 | 35.02878383 | 0.9751 | p=0.001 |
| **Biomarker5** | 39.488811 | 38.46759211 | 36.606635 | 36.0889715 | 0.9602 | p=0.025 |
| **Biomarker7** | 36.16596475 | 35.05148847 | 34.25068653 | 33.0491665 | 0.9947 | p=0.05 |
| **Biomarker8** | 36.735195 | 36.95192892 | 37.1526323 | 37.85009331 | 0.9896 | N.S. |
| **Biomarker9** | 36.76428317 | 36.59214156 | 36.82117593 | 36.59705733 | 0.0909 | N.S. |
| **Biomarker12** | NA | 38.52373733 | 38.38771118 | 37.69109103 | 0.8687 | N.S. |
| **Biomarker13** | 33.18354767 | 30.9445344 | 34.15470365 | 33.03454162 | 0.0697 | N.S. |
| **Biomarker14** | 36.77054367 | 35.119541 | 36.29562971 | 35.17122448 | 0.3125 | N.S. |

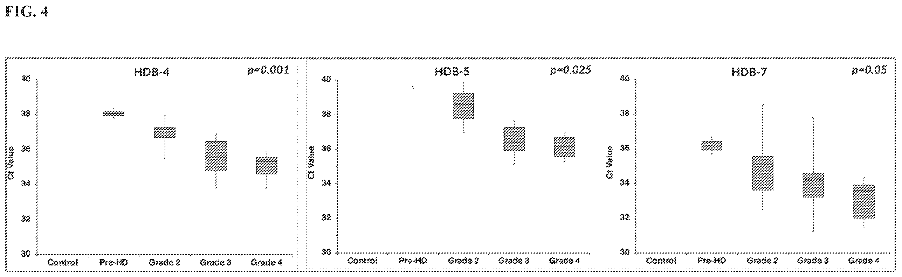

Binning Ct values according to disease grade showed that 3 of the 8 small RNA biomarkers computationally predicted to increase with disease progression, had statistically significant positive correlations with abundance using a 4-way ANOVA test (HDB-4, p=0.001; HDB-5, p=0.025; HDB-7, p=0.05), as shown in FIG. 4. Even though HDB-8 and HDB-12 displayed significant R², they did not have statistically significant ANOVA scores (p>0.05) and thus the data is not shown in FIG. 4

### Secondary Validation in Cerebrospinal Fluid (CSF)

Cerebrospinal fluid samples from 60 samples collected through the PREDICT-HD Study (See, Paulsen JS, Hayden M, Stout JC, Langbehn DR, et al. Preparing for preventative clinical trials: the Predict-HD study. Arch Neurol. 2006 Jun;63(6):883-90. PMID: 167698) were used for secondary validation. Sample set was made up of 15 Familial Controls (not carrying a CAG repeat expansion), 10 CAP Low, 10 CAP Medium, 10 CAP High, and 15 Huntington's samples. CAP score (CAG-age product) is a predictive measure used to gauge when an individual harboring a CAG repeat expansion is likely to display symptomatic onset of Huntington's Disease taking into account age and CAG length.

Total RNA was isolated from 100uL of sample material using QIAzol Lysis Reagent and purified using miRNeasy MinElute Cleanup columns (Qiagen Sciences Inc). Total RNA concentration was measured using a NanoDrop 8000, however all samples were below the limit of detection. Thus, 10uL of each sample were used for RT-qPCR validation, as described (above).

The majority of small RNAs tested in CSF (16 of 20) failed to score above the 39.999999 threshold causing almost all of the samples to drop from statistical analysis. Without wishing to be bound by theory, it is hypothesized that this was due to the significantly limited amount of CSF available for testing (100uL per sample). However, the ability to detect signal in as little as 100uL is a positive and encouraging sign that these RNAs are present in CSF and can be detected but indicates that a greater volume of sample is needed for further validation. Based on previous analysis and experience with small RNA RT-qPCR, it is posited that 1mL of CSF per sample along with a 12-cycle preamplification would be sufficient to detect these small RNAs.

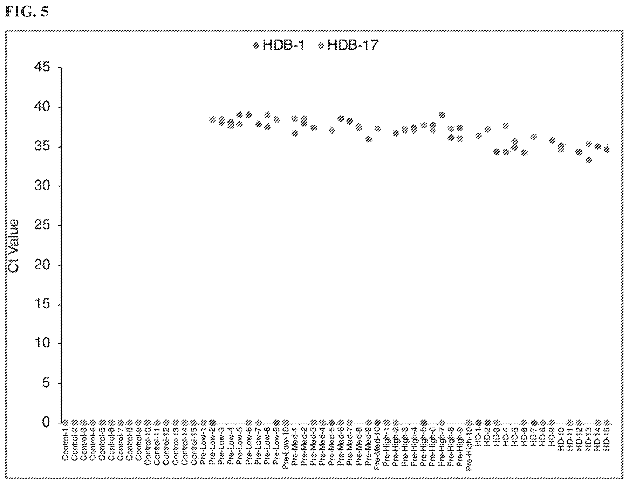

The results found that 2 of the 18 small RNA biomarkers (HDB-1 and HDB-17) selected for validation had enough Ct values within the limits of detection to perform statistical analysis. HDB-1 and HDB-17 were exclusively present in Pre-HD and HD samples, as depicted in FIG. 5.

An analysis was conducted of the Specificity and Sensitivity of small RNA biomarkers in CSF:

| | **Specificity** | **Pre-Low** | **Pre-Med** | **Pre-High** | **HD** |
|---|---|---|---|---|---|
| **HDB-1** | 100% | 60% | 70% | 70% | 67% |
| **HDB-17** | 100% | 60% | 50% | 60% | 47% |
| **Panel of 2** | 100% | 80% | 90% | 80% | 87% |

Additionally, Pearson Correlation analysis for binary small RNA predictors in CSF showed correlation coefficients of >0.8000. Mean Ct values for each biomarker were calculated per CAP_{D}. Mean values were plotted against CAP_{D} and Linear Regression Coefficients (R²) were calculated for each biomarker. Statistical significance of the variance between CAP_{D} Groups was determined by ANOVA test with 4 degrees of freedom:

| | **pre-Low** | **pre-Med** | **pre-High** | **HD** | **R²** | **ANOVA** |
|---|---|---|---|---|---|---|
| **HDB-1** | 38.2372237 | 37.4446887 | 37.3206643 | 34.5406215 | 0.8032 | p=0.001 |
| **HDB-17** | 38.2519647 | 37.7736664 | 37.004843 | 36.1409167 | 0.9847 | p=0.001 |

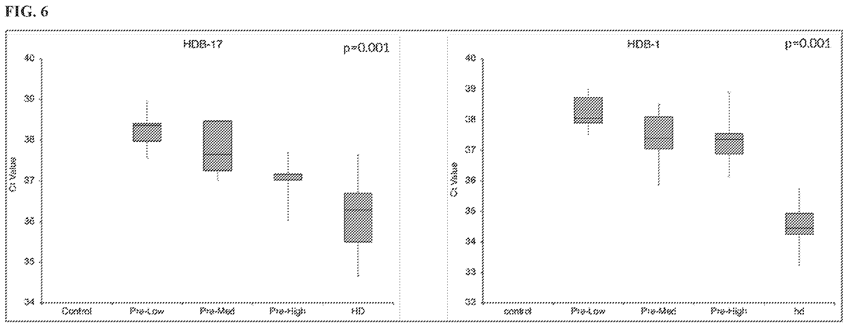

Binning Ct values according to CAP_{D} Group showed that both HDB-1 and HDB-17 had statistically significant increases correlated with CAP_{D} Group by ANOVA test, as shown in FIG. 6.

### REFERENCES

1. Hoss AG, Labadorf A, Latourelle JC, Kartha VK et al. miR-10b-5p expression in Huntington's disease brain relates to age of onset and the extent of striatal involvement. BMC Med Genomics 2015 Mar 1;8:10. PMID: 25889241
2. Hoss AG, Labadorf A, Beach TG, Latourelle JC et al. microRNA Profiles in Parkinson's Disease Prefrontal Cortex. Front Aging Neurosci 2016;8:36. PMID: 26973511
3. Santa-Maria I, Alaniz ME, Renwick N, Cela C et al. Dysregulaiton of microRNA-219 promotes neurodegeneration through post-transcriptional regulation of tau. J Clin Invest 2015 Feb;125(2):681-6. PMID: 25574843
4. Hébert SS, Wang WX, Zhu Q, Nelson PT. A study of small RNAs from cerebral neocortex of pathology-verified Alzheimer's Disease, Dementia with Lewy Bodies, Hippocampal Sclerosis, Frontotemporal Lobar Dementia, and non-demented human controls. J Alzheimers Dis 2013;35(2):335-48. PMID: 23403535
5. Segrelles C, Garcia-Escudero R, Garin MI, Aranda JF et al. Akt signaling leads to stem cell activation and promotes tumor development in epidermis. Stem Cells 2014 Jul;32(7):1917-28. PMID: 24504902
6. Pantazatos SP, Huang YY, Rosoklija GB, Dwork AJ, et al. Whole-transcriptome brain expression and exon-usage profiling in major depression and suicide: evidence for altered glial, endothelial and ATPase activity. Mol Psychiatry 2017 May;22(5):760-773. PMID: 27528462
7. Burgos K, Malenica I, Metpally R, Courtright A, et al. Profiles of extracellular miRNA in cerebrospinal fluid and serum from patients with Alzheimer's and Parkinson's diseases correlate with disease status and features of pathology. PLoS One. 2014; 9(5):e94839. PMID: 2479736
8. Juzenas S, Venkatesh G, Hübenthal M, Hoeppner MP, et al. A comprehensive, cell specific microRNA catalogue of human peripheral blood. Nucleic Acids Res 2017 Sep 19;45(16):9290-9301. PMID: 28934507
9. Klaas E, Max A, Bertram K, Akat KM, Bogardus K, et al. Human plasma and serum extracellular small RNA reference profiles and their clinical utility. Proc Natl Acad Sci U S A. 2018 Jun5;115(23) PMCID: PMC6003356
10. Leidinger P, Backes C, Deutscher S, Schmitt K, et al. A blood based 12-miRNA signature of Alzheimer disease patients. Genome Biol 2013 Jul 29;14(7):R78. PMID: 23895045
11. Kozomara A, Griffiths-Jones S. miRBase: annotating high confidence microRNAs using deep sequencing data. NAR 2014 43:D68-D73. PMID: 24275495
12. Vonstattel JP, Myers RH, Stevens TJ, Ferrante RJ, et al. Neuropathological classification of Huntington's Disease. J Neuropathol Exp Neurol. 1985 Nov;44(6):559-77. PMID: 2932539
13. Paulsen JS, Hayden M, Stout JC, Langbehn DR, et al. Preparing for preventative clinical trials: the Predict-HD study. Arch Neurol. 2006 Jun;63(6):883-90. PMID: 1676987

**Table 1A. Huntington's disease cohort for biomarker discovery.**

| **Patient** | **BU_ID** | | **PMI** | **Death** | **RIN** | **Gender** | **Onset** | **Duration** | **CAG** | **Grade** | **Striatal** | **Cortical** |
|---|---|---|---|---|---|---|---|---|---|---|---|---|
| SRR1759274 | H_1104 | PRE-HD | 22.4 | 86 | 7.2 | 2 | NA | NA | 42 | 0 | NA | NA |
| SRR1759275 | H_1105 | PRE-HD | 33.6 | 49 | 8.1 | 1 | NA | NA | 42 | 0 | NA | NA |
| SRR1759262 | H_0656 | HD | NA | 68 | 6 | 1 | 60 | 8 | 42 | 2 | 1.43 | 0.48 |
| SRR1759270 | H_0723 | HD | 9.4 | 79 | 7.9 | 1 | 70 | 9 | 40 | 2 | 1.44 | 0.79 |
| SRR1759260 | H_0513 | HD | 21.2 | 68 | 7.6 | 1 | 58 | 10 | 42 | 2 | 1.97 | 0.74 |
| SRR1759266 | H_0689 | HD | 23.4 | 68 | 6.4 | 2 | 50 | 18 | 43 | 2 | 1.52 | 0.48 |
| SRR1759249 | H_0002 | HD | 5.8 | 69 | 7.5 | 1 | 63 | 6 | 41 | 3 | 2.64 | 1.08 |
| SRR1759264 | H_0658 | HD | 11.0 | 48 | 7.8 | 1 | 42 | 6 | 44 | 3 | 2.41 | 0.98 |
| SRR1759269 | H_0709 | HD | 7.1 | 51 | 8.1 | 1 | 45 | 6 | 45 | 3 | 2.81 | 1.49 |
| SRR1759248 | H_0001 | HD | 37.3 | 55 | 7.1 | 1 | 44 | 11 | 45 | 3 | 2.66 | 0.92 |
| SRR1759261 | H_0539 | HD | 14.5 | 54 | 6.5 | 1 | 42 | 12 | 45 | 3 | 2.13 | 0.40 |
| SRR1759254 | H_0008 | HD | 21.3 | 43 | 7.4 | 1 | 28 | 15 | 49 | 3 | 2.70 | 1.70 |
| SRR1759272 | H_0740 | HD | 13.6 | 75 | 6.4 | 1 | 60 | 15 | 42 | 3 | 2.62 | 2.36 |
| SRR1759257 | H_0012 | HD | 12.8 | 68 | 6 | 1 | 52 | 16 | 42 | 3 | 2.66 | 1.08 |
| SRR1759253 | H_0007 | HD | 8.0 | 72 | 8.5 | 1 | 55 | 17 | 41 | 3 | 2.59 | 0.85 |
| SRR1759258 | H_0013 | HD | 25.1 | 57 | 6.1 | 1 | 40 | 17 | 49 | 3 | 2.91 | 1.49 |
| SRR1759268 | H_0700 | HD | 15.7 | 50 | 8 | 1 | 33 | 17 | 47 | 3 | 2.74 | 1.20 |
| SRR1759250 | H_0003 | HD | 20.5 | 71 | 7 | 1 | 52 | 19 | 43 | 3 | 2.43 | 1.71 |
| SRR1759265 | H_0681 | HD | 19.1 | 69 | 7 | 1 | 50 | 19 | 42 | 3 | 2.48 | 1.09 |
| SRR1759255 | H_0009 | HD | 3.7 | 68 | 7.8 | 1 | 45 | 23 | 42 | 3 | 2.67 | 1.70 |
| SRR1759256 | H_0010 | HD | 6.2 | 59 | 8.3 | 1 | 35 | 24 | 46 | 3 | 2.62 | 1.20 |
| SRR1759252 | H_0006 | HD | NA | 40 | 6.2 | 1 | 34 | 6 | 51 | 4 | 3.52 | 1.43 |
| SRR1759259 | H_0014 | HD | 10.6 | 48 | 7.3 | 1 | 38 | 10 | 45 | 4 | 3.60 | 1.62 |
| SRR1759273 | H_0750 | HD | 16.2 | 53 | 6 | 1 | 38 | 15 | 48 | 4 | 3.26 | 1.01 |
| SRR1759267 | H_0695 | HD | 16.2 | 55 | 7.9 | 1 | 36 | 19 | 45 | 4 | 3.58 | 2.06 |
| SRR1759251 | H_0005 | HD | 19.2 | 48 | 6.9 | 1 | 25 | 23 | 48 | 4 | 3.82 | 1.94 |
| SRR1759271 | H_0726 | HD | 14.8 | 50 | 9.2 | 1 | 27 | 23 | 48 | 4 | 3.60 | 1.20 |
| SRR1759263 | H_0657 | HD | 24.3 | 61 | 8.1 | 1 | 36 | 25 | 45 | 4 | 3.29 | 1.60 |
| **AVERAGE** | | HD | 15.7 ± 7.7 | 68 | 7.3 ± 0.9 | NA | 44.5 ± 11.8 | 15.0 ± 6.1 | 44.6 ± 2.9 | 3.1 ± 0.7 | 2.70 ± 0.65 | 1.25 ± 0.50 |
| | | PRE-HD | 28.0 ± 7.9 | 68 | 7.7 ± 0.6 | NA | NA | NA | 42.0 10 | 0 ± 0 | NA | NA |

**Table 1B. Healthy control cohort for HD biomarker discovery. No data were collected for dementia, motor onset and duration. All patients were under controlled condition.**

| **Patient ID** | BU_ID | PMI | Death | RIN |
|---|---|---|---|---|
| SRR1759212 | C_0002 | 2 | 73 | 6.3 |
| SRR1759213 | C_0003 | 2 | 91 | 7 |
| SRR1759214 | C_0004 | 2 | 82 | 8 |
| SRR1759215 | C_0005 | 2 | 97 | 8 |
| SRR1759216 | C_0006 | 5 | 86 | 8.5 |
| SRR1759217 | C_0008 | 2 | 91 | 6.7 |
| SRR1759218 | C_0009 | 3 | 81 | 7.9 |
| SRR1759219 | C_0010 | 2 | 79 | 6.5 |
| SRR1759220 | C_0011 | 2 | 63 | 7.7 |
| SRR1759221 | C_0012 | 19 | 66 | 7.1 |
| SRR1759222 | C_0013 | 15 | 69 | 7.8 |
| SRR1759223 | C_0014 | 21 | 79 | 8 |
| SRR1759224 | C_0015 | 10 | 61 | 8.2 |
| SRR1759225 | C_0016 | 20 | 58 | 8.4 |
| SRR1759226 | C_0017 | 21 | 70 | 8.2 |
| SRR1759227 | C_0018 | 17 | 66 | 8.5 |
| SRR1759228 | C_0020 | 24 | 60 | 7.9 |
| SRR1759229 | C_0021 | 26 | 76 | 7.3 |
| SRR1759230 | C_0022 | 17 | 61 | 7.8 |
| SRR1759231 | C_0023 | 18 | 62 | 6.6 |
| SRR1759232 | C_0024 | 26 | 69 | 8.7 |
| SRR1759233 | C_0025 | 25 | 61 | 8.1 |
| SRR1759234 | C_0029 | 13 | 93 | 6.4 |
| SRR1759235 | C_0031 | 24 | 53 | 7.3 |
| SRR1759236 | C_0032 | 24 | 57 | 8.3 |
| SRR1759237 | C_0033 | 15 | 43 | 7.5 |
| SRR1759238 | C_0034 | 14 | 71 | 7.8 |
| SRR1759239 | C_0035 | 21 | 46 | 7.6 |
| SRR1759240 | C_0036 | 17 | 40 | 7.5 |
| SRR1759241 | C_0037 | 28 | 44 | 8.3 |
| SRR1759242 | C_0038 | 20 | 57 | 7.7 |
| SRR1759243 | C_0039 | 15 | 80 | 7.3 |
| SRR1759244 | C_0047 | 3 | 75 | 7.1 |
| SRR1759245 | C_0049 | 4 | 76 | 8.4 |
| SRR1759246 | C_0070 | 19 | 68 | 6.3 |
| SRR1759247 | C_0087 | 19 | 64 | 8.7 |
| | | 14.4 ± 8.8 | 68.6 ± 14.3 | 7.7 ± 0.7 |

**Table 1C. Parkinson's disease cohort as a control for HD biomarker discovery.**

| **Patient** | **BU_ID** | **Dementia** | **Motor Onset** | **Duration** | **Age at Death** | **PMI** | **RIN** | **Sex** |
|---|---|---|---|---|---|---|---|---|
| SRR2353417 | P_0003 | 1 | 72 | 2 | 74 | 3 | 7.7 | 1 |
| SRR2353418 | P_0006 | 1 | NA | NA | 83 | 2 | 6.85 | 1 |
| SRR2353419 | P_0012 | 0 | 69 | 11 | 80 | 2 | 8.5 | 1 |
| SRR2353420 | P_0013 | 1 | 79 | 4 | 83 | 2 | 8.05 | 1 |
| SRR2353421 | P_0014 | 0 | 55 | 25 | 80 | 2 | 7.7 | 1 |
| SRR2353422 | P_0015 | 1 | 80 | 4 | 84 | 2 | 7.2 | 1 |
| SRR2353423 | P_0016 | 1 | 85 | 3 | 88 | 2 | 7.5 | 1 |
| SRR2353424 | P_0018 | 0 | 73 | 8 | 81 | 2 | 6.4 | 1 |
| SRR2353425 | P_0019 | 0 | 73 | 4 | 77 | 4 | 7.2 | 1 |
| SRR2353426 | P_0020 | 0 | 59 | 5 | 64 | 4 | 8 | 1 |
| SRR2353427 | P_0021 | 1 | NA | NA | 85 | 3 | 8.2 | 1 |
| SRR2353428 | P_0022 | 0 | NA | NA | 94 | 9 | 7.1 | 1 |
| SRR2353429 | P_0023 | 1 | 60 | 20 | 80 | 27 | 7.9 | 1 |
| SRR2353430 | P_0026 | 0 | 65 | 20 | 85 | 16 | 7.2 | 1 |
| SRR2353431 | P_0027 | 0 | NA | NA | 75 | 7 | 8 | 1 |
| SRR2353432 | P_0028 | 0 | NA | NA | 74 | 15 | 6.2 | 1 |
| SRR2353433 | P_0029 | 0 | 72 | 17 | 89 | 31 | 6.8 | 1 |
| SRR2353434 | P_0030 | 0 | 55 | 11 | 66 | 11 | 8.2 | 1 |
| SRR2353435 | P_0031 | 0 | NA | NA | 65 | 8 | 8 | 1 |
| SRR2353436 | P_0033 | 0 | NA | NA | 85 | 19 | 5.5 | 1 |
| SRR2353437 | P_0034 | 1 | 49 | 15 | 64 | 4 | 7.8 | 1 |
| SRR2353438 | P_0063 | 0 | 53 | 11 | 64 | 1 | 6.1 | 1 |
| SRR2353439 | P_0126 | 1 | 61 | 14 | 75 | 30 | 6.9 | 1 |
| SRR2353440 | P_0130 | 1 | 62 | 6 | 68 | 18 | 7.3 | 1 |
| SRR2353441 | P_0132 | 1 | 80 | 15 | 95 | 16 | 7 | 1 |
| SRR2353442 | P_0133 | 0 | 66 | 8 | 74 | 23 | 7.5 | 1 |
| SRR2353443 | P_0134 | 0 | 63 | 5 | 68 | 23 | 7.9 | 1 |
| SRR2353444 | P_0135 | 0 | 66 | 13 | 79 | 12 | 6.9 | 1 |
| SRR2353445 | P_0136 | 0 | NA | NA | 70 | 25 | 6.7 | 1 |
| Average | | NA | 66.5 ± 9.8 | 10.5 ± 6.5 | 77.6 ± 9.0 | 11.1 ± 9.7 | 7.3 ± 0.7 | NA |

**Table 2A. Disease Specific Biomarkers for Huntington's Disease**

| **Seq. ID** | **sRNA Type** | **Sequence** | **Total Reads** | **Frequency (Sensitivity)** | **Specificity** | **p-value in Discovery set** | |
|---|---|---|---|---|---|---|---|
| **1** | iso-miR-10b | AACCCTGTAGAACCGAATTTGTG | 444.84 | 50.00% | 100% | 5.42E-09 | |
| **2** | iso-miR-10b | CACCCTGTAGAACCGAATTTGTG | 349.34 | 42.31% | 100% | 1.63E-07 | |
| **3** | iso-miR-196a-2 | TAGGTAGTTTCATGTTGTTGGGAA | 258.61 | 38.46% | 100% | 8.26E-07 | |
| **4** | iso-miR-10b | TACCGTGTAGAACCGAATTTGTG | 501.48 | 34.62% | 100% | 3.99E-06 | |
| **5** | iso-SNORD115-5 | GATCGATGATGAGAACCTTATATTGTCCTGCAGAGAA | 333.01 | 34.62% | 100% | 3.99E-06 | |
| **6** | iso-miR-196a-2 | TAGGTAGTTTCATGTTGTTGGGT | 293.85 | 34.62% | 100% | 3.99E-06 | |
| **7** | iso-miR-10b | TACCCTGTAGAATCGAATTTG | 276.45 | 34.62% | 100% | 3.99E-06 | |
| **8** | iso-miR-10b | ACCCTGTAGAACCGAAATTGTGA | 254.04 | 34.62% | 100% | 3.99E-06 | |
| **9** | iso-miR-10b | GCCCTGTAGAACCGAATTTGT | 246.37 | 34.62% | 100% | 3.99E-06 | |
| **10** | iso-miR-10b | TACCCTGTAGAACCGAATTTGTGTGG | 228.88 | 34.62% | 100% | 3.99E-06 | |
| **11** | iso-miR-196a-2 | TAGGTAGTTTCATGTTGTTGGGAT | 227.4 | 34.62% | 100% | 3.99E-06 | |
| **12** | iso-miR-10b | ACCCTGTAGAACTGAATTTGTGT | 293.61 | 30.77% | 100% | 1.84E-05 | |
| **13** | iso-miR-10b | TACCCTGTAGAACCGAATTTGAG | 231.3 | 30.77% | 100% | 1.84E-05 | |
| **14** | iso-miR-10b | ATGAAAAGAACTTTGAAAAGAG | 197.35 | 30.77% | 100% | 1.84E-05 | |
| **15** | novel sRNA | ATTACTCCTGCCATCATGACCCTTGGCCATAAT | 193.28 | 30.77% | 100% | 1.84E-05 | |
| **16** | iso-miR-10b | TACCCTGTAGAACCGAATTTGTAG | 192.85 | 30.77% | 100% | 1.84E-05 | |
| **17** | iso-miR-10b | TACGTCGCCCTGGACTTCGAGCAGGAGA | 176.14 | 30.77% | 100% | 1.84E-05 | |
| **18** | iso-miR-10b | ACACTGTAGAACCGAATTTGTG | 138.53 | 30.77% | 100% | 1.84E-05 | |
| **19** | iso-miR-10b | TACCCTGTCGAACCGAATTTGT | 808.19 | 26.92% | 100% | 8.13E-05 | |
| **20** | iso-miR-10b | TACCCTGTAGCACCGAATTTGTGA | 421.48 | 26.92% | 100% | 8.13E-05 | |
| **21** | iso-miR-10b | TACCCTGTAGAACCGAATTTTTT | 218.01 | 26.92% | 100% | 8.13E-05 | |
| **22** | iso-miR-125b | CCCGTGGACAAGTCAGGCTCTTGGGACCTT | 210.27 | 26.92% | 100% | 8.13E-05 | |
| **23** | iso-miR-148a | TCAGTGCACTACAGAACTTTTA | 160.39 | 26.92% | 100% | 8.13E-05 | |
| **24** | iso-miR-10b | ACCCTGTAGAACCGAATTTATG | 277.38 | 23.08% | 100% | 3.45E-04 | |
| **25** | iso-miR-10b | ACCCTGTAGAACCGAGTTTGTG | 231.76 | 23.08% | 100% | 3.45E-04 | |
| **26** | iso-SNORD104 | TTAAAGCACGTGTTAGACTG | 137.04 | 23.08% | 100% | 3.45E-04 | |
| **27** | novel sRNA | TACCCATTGCATATCGGAATTGT | 115.29 | 23.08% | 100% | 3.45E-04 | |
| **28** | iso-SNORD2 | TTCGGGACTGACCTGAAATGAAGAGAATACTCATTGCC GA | 101.15 | 23.08% | 100% | 3.45E-04 | |
| **29** | iso-miR-5188 | AATCGGACCCATTTAAACCGG | 95.19 | 23.08% | 100% | 3.45E-04 | |

| **Seq.ID** | **SRR1759260** | **SRR1759262** | **SRR1759266** | **SRR1759270** | **SRR1759248** | **SRR1759249** | **SRR1759250** |
|---|---|---|---|---|---|---|---|
| **1** | 0 | 0 | 0 | 0 | 0 | 0 | 17.48 |
| **2** | 0 | 0 | 0 | 0 | 0 | 0 | 17.48 |
| **3** | 0 | 14.36 | 0 | 0 | 52.32 | 0 | 0 |
| **4** | 0 | 0 | 0 | 0 | 0 | 0 | 0 |
| **5** | 0 | 0 | 0 | 0 | 0 | 42.14 | 17.48 |
| **6** | 0 | 0 | 0 | 12.08 | 17.44 | 0 | 0 |
| **7** | 0 | 0 | 0 | 0 | 17.44 | 0 | 17.48 |
| **8** | 0 | 0 | 14.98 | 0 | 0 | 21.07 | 0 |
| **9** | 0 | 0 | 0 | 0 | 17.44 | 42.14 | 0 |
| **10** | 13.77 | 0 | 0 | 0 | 0 | 0 | 0 |
| **11** | 0 | 0 | 0 | 0 | 17.44 | 0 | 34.96 |
| **12** | 0 | 0 | 0 | 0 | 0 | 0 | 0 |
| **13** | 0 | 0 | 0 | 0 | 17.44 | 0 | 17.48 |
| **14** | 13.77 | 0 | 0 | 0 | 17.44 | 0 | 34.96 |
| **15** | 0 | 0 | 0 | 0 | 17.44 | 0 | 0 |
| **16** | 0 | 0 | 0 | 0 | 0 | 0 | 0 |
| **17** | 0 | 0 | 0 | 12.08 | 0 | 21.07 | 0 |
| **18** | 0 | 0 | 14.98 | 0 | 0 | 0 | 0 |
| **19** | 0 | 0 | 0 | 0 | 17.44 | 0 | 0 |
| **20** | 0 | 0 | 0 | 0 | 0 | 0 | 0 |
| **21** | 0 | 0 | 0 | 0 | 0 | 0 | 0 |
| **22** | 0 | 0 | 0 | 0 | 0 | 42.14 | 34.96 |
| **23** | 0 | 0 | 0 | 0 | 0 | 0 | 0 |
| **24** | 0 | 0 | 0 | 0 | 0 | 0 | 0 |
| **25** | 13.77 | 0 | 0 | 0 | 0 | 0 | 17.48 |
| **26** | 0 | 14.36 | 0 | 0 | 0 | 0 | 0 |
| **27** | 0 | 14.36 | 14.98 | 12.08 | 0 | 0 | 0 |
| **28** | 13.77 | 14.36 | 0 | 0 | 17.44 | 0 | 17.48 |
| **29** | 0 | 14.36 | 0 | 12.08 | 17.44 | 0 | 0 |
| **# of biomarkers per patient** | 4 | 5 | 3 | 4 | 11 | 5 | 10 |
| **% coverage** | 13.8% | 17.2% | 10.3% | 13.8% | 37.9% | 17.2% | 34.5% |

| **Seq. ID** | **SRR1759253** | **SRR1759254** | **SRR1759255** | **SRR1759256** | **SRR1759257** | **SRR1759258** | **SRR1759261** |
|---|---|---|---|---|---|---|---|
| **1** | 27.22 | 38.54 | 42.86 | 41.36 | 20.91 | 23.61 | 0 |
| **2** | 0 | 19.27 | 0 | 20.68 | 0 | 0 | 0 |
| **3** | 13.61 | 57.81 | 0 | 20.68 | 0 | 0 | 0 |
| **4** | 0 | 96.35 | 85.72 | 0 | 0 | 0 | 0 |
| **5** | 0 | 38.54 | 85.72 | 0 | 0 | 0 | 0 |
| **6** | 0 | 0 | 42.86 | 0 | 0 | 23.61 | 0 |
| **7** | 0 | 19.27 | 0 | 0 | 0 | 0 | 0 |
| **8** | 13.61 | 19.27 | 0 | 0 | 0 | 0 | 0 |
| **9** | 13.61 | 96.35 | 0 | 0 | 0 | 0 | 0 |
| **10** | 0 | 19.27 | 0 | 0 | 0 | 0 | 0 |
| **11** | 0 | 57.81 | 21.43 | 0 | 0 | 0 | 0 |
| **12** | 0 | 38.54 | 0 | 0 | 0 | 47.22 | 0 |
| **13** | 0 | 0 | 0 | 0 | 0 | 0 | 0 |
| **14** | 0 | 19.27 | 0 | 0 | 0 | 23.61 | 0 |
| **15** | 27.22 | 0 | 21.43 | 0 | 0 | 47.22 | 0 |
| **16** | 0 | 0 | 0 | 20.68 | 0 | 0 | 0 |
| **17** | 0 | 0 | 0 | 0 | 0 | 47.22 | 0 |
| **18** | 0 | 0 | 21.43 | 20.68 | 0 | 0 | 18.38 |
| **19** | 0 | 19.27 | 0 | 0 | 0 | 23.61 | 0 |
| **20** | 0 | 0 | 0 | 165.44 | 20.91 | 23.61 | 0 |
| **21** | 0 | 0 | 21.43 | 0 | 0 | 0 | 18.38 |
| **22** | 13.61 | 0 | 21.43 | 0 | 41.82 | 0 | 0 |
| **23** | 0 | 0 | 0 | 0 | 0 | 0 | 18.38 |
| **24** | 0 | 0 | 0 | 0 | 0 | 0 | 18.38 |
| **25** | 0 | 0 | 21.43 | 0 | 0 | 0 | 0 |
| **26** | 0 | 0 | 0 | 0 | 0 | 0 | 0 |
| **27** | 0 | 0 | 0 | 0 | 0 | 0 | 0 |
| **28** | 0 | 19.27 | 0 | 0 | 0 | 0 | 0 |
| **29** | 13.61 | 0 | 0 | 20.68 | 0 | 0 | 0 |
| **# of biomarkers per patient** | 7 | 14 | 10 | 7 | 3 | 8 | 4 |
| **% coverage** | 24.1% | 48.3% | 34.5% | 24.1% | 10.3% | 27.6% | 13.8% |

| **Seq. ID** | **SRR1759264** | **SRR1759265** | **SRR1759268** | **SRR1759269** | **SRR1759272** | **SRR1759251** | **SRR1759252** |
|---|---|---|---|---|---|---|---|
| **1** | 0 | 15.1 | 0 | 0 | 0 | 83.52 | 43.65 |
| **2** | 0 | 15.1 | 0 | 34.04 | 0 | 41.76 | 29.1 |
| **3** | 0 | 0 | 0 | 17.02 | 0 | 20.28 | 29.1 |
| **4** | 0 | 0 | 18.32 | 0 | 17.44 | 0 | 43.65 |
| **5** | 12.64 | 0 | 0 | 0 | 17.44 | 83.52 | 0 |
| **6** | 12.64 | 0 | 0 | 0 | 0 | 83.52 | 0 |
| **7** | 25.28 | 0 | 36.64 | 0 | 0 | 41.76 | 0 |
| **8** | 25.28 | 0 | 36.64 | 0 | 0 | 20.28 | 72.75 |
| **9** | 12.64 | 15.1 | 0 | 0 | 0 | 20.28 | 14.55 |
| **10** | 12.64 | 15.1 | 36.64 | 17.02 | 0 | 0 | 0 |
| **11** | 12.64 | 0 | 0 | 0 | 0 | 0 | 29.1 |
| **12** | 25.28 | 0 | 54.96 | 17.02 | 0 | 0 | 0 |
| **13** | 63.2 | 0 | 0 | 17.02 | 0 | 20.28 | 0 |
| **14** | 0 | 0 | 18.32 | 0 | 0 | 0 | 0 |
| **15** | 0 | 0 | 0 | 17.02 | 0 | 20.28 | 14.55 |
| **16** | 0 | 15.1 | 18.32 | 0 | 34.88 | 0 | 0 |
| **17** | 12.64 | 0 | 0 | 0 | 17.44 | 0 | 0 |
| **18** | 0 | 0 | 0 | 0 | 0 | 0 | 14.55 |
| **19** | 0 | 0 | 54.96 | 0 | 0 | 0 | 0 |
| **20** | 0 | 0 | 18.32 | 0 | 0 | 0 | 0 |
| **21** | 0 | 15.1 | 0 | 0 | 17.44 | 41.76 | 0 |
| **22** | 0 | 0 | 0 | 0 | 0 | 41.76 | 14.55 |
| **23** | 0 | 0 | 36.64 | 0 | 17.44 | 0 | 0 |
| **24** | 0 | 0 | 0 | 17.02 | 0 | 0 | 0 |
| **25** | 0 | 0 | 0 | 0 | 0 | 0 | 0 |
| **26** | 12.64 | 0 | 18.32 | 0 | 34.88 | 41.76 | 0 |
| **27** | 25.28 | 0 | 0 | 34.04 | 0 | 0 | 14.55 |
| **28** | 0 | 0 | 0 | 0 | 0 | 0 | 0 |
| **29** | 0 | 0 | 0 | 17.02 | 0 | 0 | 0 |
| **# of biomarkers per patient** | 12 | 6 | 11 | 9 | 7 | 13 | 11 |
| **% coverage** | 41.4% | 20.7% | 37.9% | 31.0% | 24.1% | 44.8% | 37.9% |

| **Seq. ID** | **SRR1759259** | **SRR1759263** | **SRR1759267** | **SRR1759271** | **SRR1759273** | | |
|---|---|---|---|---|---|---|---|
| **1** | 57.16 | 0 | 0 | 14.6 | 18.83 | | |
| **2** | 85.74 | 0 | 15.08 | 14.6 | 56.49 | | |
| **3** | 0 | 0 | 0 | 14.6 | 18.83 | | |
| **4** | 14.26 | 104.5 | 19.04 | 102.2 | 0 | | |
| **5** | 0 | 20.93 | 0 | 14.6 | 0 | | |
| **6** | 0 | 41.86 | 45.24 | 14.6 | 0 | | |
| **7** | 28.58 | 0 | 75.4 | 14.6 | 0 | | |
| **8** | 0 | 0 | 30.16 | 0 | 0 | | |
| **9** | 14.26 | 0 | 0 | 0 | 0 | | |
| **10** | 42.87 | 0 | 15.08 | 0 | 56.49 | | |
| **11** | 14.26 | 20.93 | 0 | 0 | 18.83 | | |
| **12** | 14.26 | 20.93 | 75.4 | 0 | 0 | | |
| **13** | 14.26 | 62.79 | 0 | 0 | 18.83 | | |
| **14** | 0 | 41.86 | 0 | 28.12 | 0 | | |
| **15** | 0 | 0 | 0 | 28.12 | 0 | | |
| **16** | 28.58 | 41.86 | 0 | 14.6 | 18.83 | | |

| **Seq. ID** | **SRR1759259** | | **SRR1759263** | **SRR1759267** | **SRR1759271** | **SRR1759273** | |
|---|---|---|---|---|---|---|---|
| **17** | 0 | | 20.93 | 30.16 | 14.6 | 0 | |
| **18** | 0 | | 0 | 15.08 | 14.6 | 18.83 | |
| **19** | 0 | | 230.23 | 316.68 | 146 | 0 | |
| **20** | 157.19 | | 20.93 | 15.08 | 0 | 0 | |
| **21** | 28.58 | | 0 | 0 | 0 | 75.32 | |
| **22** | 0 | | 0 | 0 | 0 | 0 | |
| **23** | 14.26 | | 20.93 | 15.08 | 0 | 37.66 | |
| **24** | 0 | | 41.86 | 8 | 116.8 | 75.32 | |
| **25** | 14.26 | | 104.5 | 60.32 | 0 | 0 | |
| **26** | 0 | | 0 | 15.08 | 0 | 0 | |
| **27** | 0 | | 0 | 0 | 0 | 0 | |
| **28** | 0 | | 0 | 0 | 0 | 18.83 | |
| **29** | 0 | | 0 | 0 | 0 | 0 | |
| **# of biomarkers per patient** | 14 | | 14 | 15 | 14 | 12 | |
| **% coverage** | 48.3% | | 48.3% | 51.7% | 48.3% | 41.4% | |

**Table 2B. Average Read Count of Disease Specific Biomarkers in Each Disease Grade**

| **Seq ID** | **sRNA Type** | **Sequence** | **Grade 2** | **Grade 3** | **Grade 4** |
|---|---|---|---|---|---|
| **1** | iso-miR-10b | AACCCTGTAGAACCGAATTTGTG | 0.00 | 13.48 | 27.35 |
| **2** | iso-miR-10b | CACCCTGTAGAACCGAATTTGTG | 0.00 | 6.50 | 30.60 |
| **3** | iso-miR-196a-2 | TAG GTAGTTTCATGTTGTTGG GAA | 3.59 | 9.85 | 10.73 |
| **4** | iso-miR-10b | TACCGTGTAGAACCGAATTTGTG | 0.00 | 13.28 | 35.96 |
| **5** | iso-SNORD115-5 | GATCGATGATGAGAACCTTATATTGTCCTGCAGAGAA | 0.00 | 13.17 | 15.51 |
| **6** | iso-miR-196a-2 | TAG GTAGTTTCATGTTGTTG GGT | 3.02 | 6.39 | 23.90 |
| **7** | iso-miR-10b | TACCCTGTAGAATCGAATTTG | 0.00 | 7.65 | 20.92 |
| **8** | iso-miR-10b | ACCCTGTAGAACCGAAATTGTGA | 3.75 | 7.76 | 16.40 |
| **9** | iso-miR-10b | GCCCTGTAGAACCGAATTTGT | 0.00 | 12.66 | 7.26 |
| **10** | iso-miR-10b | TACCCTGTAGAACCGAATTTGTGTGG | 3.44 | 7.10 | 15.56 |
| **11** | iso-miR-196a-2 | TAG GTAGTTTCATGTTGTTGG GAT | 0.00 | 9.78 | 11.77 |
| **12** | iso-miR-10b | ACCCTGTAGAACTGAATTTGTGT | 0.00 | 12.18 | 15.32 |
| **13** | iso-miR-10b | TACCCTGTAGAACCGAATTTGAG | 0.00 | 8.30 | 16.15 |
| **14** | iso-miR-10b | ATGAAAAGAACTTTGAAAAGAG | 3.44 | 8.33 | 10.50 |
| **15** | novel sRNA | ATTACTCCTGCCATCATGACCCTTGGCCATAAT | 0.00 | 9.43 | 9.74 |
| **16** | iso-miR-10b | TACCCTGTAGAACCGAATTTGTAG | 0.00 | 7.12 | 14.98 |
| **17** | iso-miR-10b | TACGTCGCCCTGGACTTCGAGCAGGAGA | 3.02 | 7.79 | 10.34 |
| **18** | iso-miR-10b | ACACTGTAGAACCGAATTTGTG | 3.75 | 5.68 | 10.13 |
| **19** | iso-miR-10b | TACCCTGTCGAACCGAATTTGT | 0.00 | 9.02 | 88.99 |
| **20** | iso-miR-10b | TACCCTGTAGCACCGAATTTGTGA | 0.00 | 15.78 | 26.65 |
| **21** | iso-miR-10b | TACCCTGTAGAACCGAATTTTTT | 0.00 | 6.73 | 20.83 |
| **22** | iso-miR-125b | CCCGTGGACAAGTCAGGCTCTTGGGACCTT | 0.00 | 11.64 | 9.79 |
| **23** | iso-miR-148a | TCAGTGCACTACAGAACTTTTA | 0.00 | 6.97 | 13.87 |
| **24** | iso-miR-10b | ACCCTGTAGAACCGAATTTATG | 0.00 | 4.91 | 33.25 |
| **25** | iso-miR-10b | ACCCTGTAGAACCGAGTTTGTG | 3.44 | 5.23 | 25.51 |
| **26** | iso-SNORD104 | TTAAAGCACGTGTTAGACTG | 3.59 | 6.93 | 10.36 |
| **27** | novel sRNA | TACCCATTGCATATCGGAATTGT | 10.36 | 6.67 | 5.19 |
| **28** | iso-SNORD2 | TTCGGGACTGACCTGAAATGAAGAGAATACTCATTGCCGA | 7.03 | 6.48 | 5.85 |
| **29** | iso-miR-5188 | AATCGGACCCATTTAAACCGG | 6.61 | 7.46 | 3.63 |

**Table 3. Grade Specific Biomarkers for Huntington's Disease**

| **Seq ID** | **sRNA Type** | **Sequence** | **Total Reads** | **Frequency (Sensitivity)** | **Specificity** | **p value of sRNA in Discovery Set** | | |
|---|---|---|---|---|---|---|---|---|
| **30** | tRNA-derived sRNA | CCCTGGTGGTCTAGTGGTTAGGATTTGG CGCTCTCACC | 209.94 | 100.00% | 100.00% | 2.34E-04 | | |
| **31** | Novel sRNA | GGCACCTTGATCATGGACTTCCTAGCCTC CAGAA | 85.38 | 100.00% | 100.00% | 2.34E-04 | | |
| **32** | Novel sRNA | GGCACCTTGATCATGGACTTCCTAGCCTC CAGAACCC | 72.93 | 100.00% | 100.00% | 2.34E-04 | | |
| **33** | Novel sRNA | TCCCTGGTCTAGTGGTTAGGATTTGG | 72.93 | 100.00% | 100.00% | 2.34E-04 | | |
| **34** | Novel sRNA | TCTTCCGGAGATGTAGCAAAACGCATGG AGTGTGTATTG | 58.66 | 100.00% | 100.00% | 2.34E-04 | | |
| **35** | Novel sRNA | AGTTCCTCCTTGTACCTCTGGTAGAATTC | 48.02 | 100.00% | 100.00% | 2.34E-04 | | |
| **36** | Novel sRNA | CCAGTACTATCTGCGGGTCACCACGG | 48.02 | 100.00% | 100.00% | 2.34E-04 | | |
| **37** | Novel sRNA | GAGCTGTAGGGCCAGCTGCCGGGCTC | 46.21 | 100.00% | 100.00% | 2.34E-04 | | |
| **38** | Novel sRNA | ATTGGTCGTGGTTGTAGTGTGTGCGAGA ATA | 46.21 | 100.00% | 100.00% | 2.34E-04 | | |
| **39** | iso-miR-24-1 | TGTCGATTGGACCCGCCCTCCGG | 35.56 | 100.00% | 100.00% | 2.34E-04 | | |
| **40** | tRNA-derived sRNA | GTCTCTGTGGCGCAATCGGTTAGCGCTT CGGCT | 69.54 | 100.00% | 100.00% | 4.29E-07 | | |
| **41** | IncRNA | CTGAGGCTGCAGGATCGCTTGAGTCCA GGAG | 55.18 | 100.00% | 100.00% | 4.29E-07 | | |
| **42** | Novel sRNA | AGAGAACCAAGCCAGAATTCTGATCCTC | 149.56 | 75.00% | 100.00% | 3.65E-05 | | |
| **43** | Novel sRNA | ATCCTAACGAACGAACGATTTGAAC | 130.45 | 75.00% | 100.00% | 3.65E-05 | | |
| **44** | Novel sRNA | GTGATGTATGCAGCTGAGGCATCCTAA CGAACGAACGAT | 128.77 | 75.00% | 100.00% | 3.65E-05 | | |
| **45** | iso-SNORD116 | TATCGATG ATG ACTTCCATATA | 85.00 | 75.00% | 100.00% | 3.65E-05 | | |
| **46** | tRNA-derived sRNA | GCATCTCGGTTCGAATCCGAGTGGCGG CACCA | 64.99 | 75.00% | 100.00% | 3.65E-05 | | |
| **47** | tRNA-derived sRNA | GCCCGGCTAGCTCAGTCGGTAGAGCAT GCACTC | 58.08 | 75.00% | 100.00% | 3.65E-05 | | |
| **48** | Novel sRNA | CGAGCTGACACTTTCCTTGGCATAGAGA ACTTGGAGTA | 53.98 | 75.00% | 100.00% | 3.65E-05 | | |
| **49** | IncRNA | ACACTTCGAACGCACTTGCGGCCCCGGGA | 43.10 | 75.00% | 100.00% | 3.65E-05 | | |
| **50** | Novel sRNA | AGTTGTCTTGAACCAGGACGGAGAGAG ACAGCCTCGGAC | 43.10 | 75.00% | 100.00% | 3.65E-05 | | |
| **51** | iso-miR-124 | TAAGGCACGCGGTGAATGCCAAAGCAT TGGTGGTTCAGTGG | 41.42 | 75.00% | 100.00% | 3.65E-05 | | |
| **52** | Novel sRNA | ATGACATTCGTCTGAGACCAGA | 40.83 | 75.00% | 100.00% | 3.65E-05 | | |
| **53** | Novel sRNA | AGCACCTGACCCCGAGGACTGG | 40.21 | 75.00% | 100.00% | 3.65E-05 | | |
| **54** | Novel sRNA | CTGCACCCCCTTCTTG GCTGT | 40.21 | 75.00% | 100.00% | 3.65E-05 | | |
| **55** | iso-miR-219a-2 | GATGTCCAGCCACAATTCTC | 40.21 | 75.00% | 100.00% | 3.65E-05 | | |
| **56** | iso-SNORD3B | TTTTCTCCTGAGCGTGAAACCGGCTTTT | 267.62 | 50.00% | 100.00% | 1.57E-03 | | |
| **57** | iso-SNORD3B | GTTTTCTCCTGAGCGTGAAACCGGCTTT | 102.41 | 50.00% | 100.00% | 1.57E-03 | | |
| **58** | tRNA-derived sRNA | CATAATCTGAAGGTCGTGAGTTCGATCC TCCCACGGGGCACCA | 256.83 | 46.67% | 100.00% | 7.95E-07 | | |
| **59** | SNORD26 | CTACGGGGATGACTTTACGAACTGAAC TCTCTCTTTCTGA | 185.58 | 46.67% | 100.00% | 7.95E-07 | | |
| **60** | IncRNA | CTAACTGATGAGCAAAGTGAGGCCCAG AGAGACGCTCAAGTCA | 200.16 | 40.00% | 100.00% | 7.51E-06 | | |
| **61** | scaRNA | CCACATGATGATACCAAGGCTGTTG | 189.01 | 40.00% | 100.00% | 7.51E-06 | | |
| **62** | SNORD116 | ATCGATGATGACTTAAAGATTTAACTAA | 270.43 | 33.33% | 100.00% | 6.46E-05 | | |
| **63** | SNORD18A | CCACTTCATTGGTCCGTGTTTCTGAACCAC | 259.14 | 33.33% | 100.00% | 6.46E-05 | | |
| **64** | SNORD27 | ACTCCATGATGAACACAAAATGATAA GCATATGGC | 248.56 | 33.33% | 100.00% | 6.46E-05 | | |
| **65** | SNORD14 | ACCAATGATGACAAATACCCGCG | 228.49 | 33.33% | 100.00% | 6.46E-05 | | |
| **66** | SNORD90 | GCCTAATGATGAATTTCATAGGGCAGA | 220.69 | 33.33% | 100.00% | 6.46E-05 | | |
| | | TTCTGAGGTGAAAATT | | | | | | |
| **67** | SNORD26 | CTACGGGGATGATTTTACGAACTGAAC TCTCTCTTTATGA | 213.50 | 33.33% | 100.00% | 6.46E-05 | | |
| **68** | iso-SNORD116 | GATCGATGATGACTTTCATAAA | 207.83 | 33.33% | 100.00% | 6.46E-05 | | |
| **69** | iso-SNORD116 | GATCGATGATGAGTCCCCTTTAAAAAC ATTCCT | 143.61 | 33.33% | 100.00% | 6.46E-05 | | |
| **70** | iso-SNORD27 | CTCAATGATGAACACAAAATGACAAG CATATGGC | 136.68 | 33.33% | 100.00% | 6.46E-05 | | |
| **71** | iso-SNORD116 | ATCGATAATGACTTAAAGATTTATCTAA | 127.95 | 33.33% | 100.00% | 6.46E-05 | | |
| **72** | iso-SNORDS | ACGGGCATGAACTAAAACTTAA | 118.77 | 33.33% | 100.00% | 6.46E-05 | | |
| **73** | Novel sRNA | AAAGCGGCTGTGCAGACATTCAATTG | 117.90 | 33.33% | 100.00% | 6.46E-05 | | |
| **74** | IncRNA | AGCCGCCTGGATACCGTAGCTAGGAA TAATGGAATAGG | 116.70 | 33.33% | 100.00% | 6.46E-05 | | |
| **75** | Novel sRNA | GAAATACAACGATGGTTTTTCATATCA TTGGTCGTGGTTGTAGTA | 115.42 | 33.33% | 100.00% | 6.46E-05 | | |
| **76** | Novel sRNA | CAGAGTGTAGCTTAACACAAAGCACCC AACTTACACTTAGTTGGG | 113.32 | 33.33% | 100.00% | 6.46E-05 | | |
| **77** | iso-SNORD115 | ATCGATGATGAGAACCTTATATTGTCC TGAAGCGAA | 112.74 | 33.33% | 100.00% | 6.46E-05 | | |
| **78** | iso-miR-196a-1 | TAGGTAGTTTCATGTTGTTGGGG | 110.47 | 33.33% | 100.00% | 6.46E-05 | | |
| **79** | SNORA57 | GAGGGAAAGGGCTCTGGCCCCC | 110.46 | 33.33% | 100.00% | 6.46E-05 | | |
| **80** | Novel sRNA | ATAGGTTTGGTCCTAGCCTTTCTG | 109.15 | 33.33% | 100.00% | 6.46E-05 | | |
| **81** | iso-SNORD2 | TCGGGACTGACCTGAAATGAAGAGAA TACTTCTTGCTGATC | 96.68 | 33.33% | 100.00% | 6.46E-05 | | |
| **82** | Novel sRNA | GATGAAACCGATATCGCCGATACGGTTGTA | 94.93 | 33.33% | 100.00% | 6.46E-05 | | |
| **83** | IncRNA | GTTTCCGTAGTGTAGTGGTTATCACCT TTTCCCTTT | 89.85 | 33.33% | 100.00% | 6.46E-05 | | |
| **84** | IncRNA | GATGGGCATGAAACTGTGGTTTGCTC | 84.76 | 33.33% | 100.00% | 6.46E-05 | | |
| | | CACCGACA | | | | | | |
| **85** | iso-miR-let-7b | AATTTCGGTTGGGTGAGGTAGTAGGT TGTGTGGTT | 83.66 | 33.33% | 100.00% | 6.46E-05 | | |
| **86** | Novel sRNA | AGTAAGGTAAGCTAAATAAGCTATCG GGACCA | 110.17 | 57.14% | 100.00% | 1.20E-05 | | |
| **87** | iso-SNORD107 | GGTTCATGATGACACAGGAGCTTGTC TGAAC | 98.65 | 57.14% | 100.00% | 1.20E-05 | | |
| **88** | iso-miR-10b | ACGCTGTAGAACCGAATTTGTGA | 69.44 | 57.14% | 100.00% | 1.20E-05 | | |
| **89** | iso-miR-532 | CATGCCTTGAGTGGAGGACCGTA | 69.44 | 57.14% | 100.00% | 1.20E-05 | | |
| **90** | Novel sRNA | TGAATCTGATAACAGAGGCTTACGACC CCTTA | 62.27 | 57.14% | 100.00% | 1.20E-05 | | |
| **91** | Novel sRNA | GGATATCAGCATATACTGTTAGT | 145.97 | 42.86% | 100.00% | 2.70E-04 | | |
| **92** | iso-SNORD115 | GTCGATGATGAGAACCTTATATTTTCCTG AAGAAGA | 73.99 | 42.86% | 100.00% | 2.70E-04 | | |
| **93** | iso-miR-10b | ACCCTGTAGATCCAAATTTGTGA | 73.67 | 42.86% | 100.00% | 2.70E-04 | | |
| **94** | iso-miR-10b | ACCCTGTAGAAACGAATTTGTGA | 72.01 | 42.86% | 100.00% | 2.70E-04 | | |
| **95** | iso-miR-let-7a-3 | TGAGGTAGTAGGTTGTATAGTTTGGTG GTGGC | 71.00 | 42.86% | 100.00% | 2.70E-04 | | |
| **96** | rRNA-derived sRNA | AATTCCGATAACGAACGAGACTCTGGC ATGCTACCTAGT | 68.85 | 42.86% | 100.00% | 2.70E-04 | | |
| **97** | iso-miR-9-2 | TCTTTGGTTATCTAGCTGTAAACA | 63.39 | 42.86% | 100.00% | 2.70E-04 | | |
| **98** | iso-miR-148a | AAAGGTCTGAGACACTCCGACT | 56.28 | 42.86% | 100.00% | 2.70E-04 | | |
| **99** | iso-SNORD115 | GTCAATGATGACAACCTTACATTGTCCT GAAGAGAGATGATGACT | 55.75 | 42.86% | 100.00% | 2.70E-04 | | |
| **100** | tRNA-derived sRNA | AACCCAGAGGTCGATGGATCGAAACCA TCCTC | 54.04 | 42.86% | 100.00% | 2.70E-04 | | |
| **101** | Novel sRNA | GGAGGGCTGAGAGGGCCCCTGTGA | 50.55 | 42.86% | 100.00% | 2.70E-04 | | |
| **102** | iso-miR-127 | TCGGAGCCGTCTGAGCTTGGCTTTA | 50.55 | 42.86% | 100.00% | 2.70E-04 | | |

| **Seq ID** | **PRE-HD** | | **Grade 2 Huntington's Disease** | | | | | |
|---|---|---|---|---|---|---|---|---|
| **-** | SRR1759274 | SRR1759275 | SRR1759260 | SRR1759262 | SRR1759266 | SRR1759270 | | |
| **30** | 10.65 | 199.29 | - | - | - | - | | |
| **31** | 10.65 | 74.73 | - | - | - | - | | |
| **32** | 10.65 | 62.28 | - | - | - | - | | |
| **33** | 10.65 | 62.28 | - | - | - | - | | |
| **34** | 21.30 | 37.37 | - | - | - | - | | |
| **35** | 10.65 | 37.37 | - | - | - | - | | |
| **36** | 10.65 | 37.37 | - | - | - | - | | |
| **37** | 21.30 | 24.91 | - | - | - | - | | |
| **38** | 21.30 | 24.91 | - | - | - | - | | |
| **39** | 10.65 | 24.91 | - | - | - | - | | |
| **40** | - | - | 13.77 | 28.72 | 14.98 | 12.08 | | |
| **41** | - | - | 13.77 | 14.36 | 14.98 | 12.08 | | |
| **42** | - | - | 13.77 | 0.00 | 14.98 | 120.82 | | |
| **43** | - | - | 55.07 | 0.00 | 14.98 | 60.41 | | |
| **44** | - | - | 41.30 | 0.00 | 14.98 | 72.49 | | |
| **45** | - | - | 41.30 | 28.72 | 14.98 | 0.00 | | |
| **46** | - | - | 13.77 | 0.00 | 14.98 | 36.25 | | |
| **47** | - | - | 13.77 | 14.36 | 29.95 | 0.00 | | |
| **48** | - | - | 27.53 | 14.36 | 0.00 | 12.08 | | |
| **49** | - | - | 13.77 | 14.36 | 14.98 | 0.00 | | |
| **50** | - | - | 13.77 | 14.36 | 14.98 | 0.00 | | |
| **51** | - | - | 0.00 | 14.36 | 14.98 | 12.08 | | |
| **52** | - | - | 13.77 | 0.00 | 14.98 | 12.08 | | |
| **53** | - | - | 13.77 | 14.36 | 0.00 | 12.08 | | |
| **54** | - | - | 13.77 | 14.36 | 0.00 | 12.08 | | |
| **55** | - | - | 13.77 | 14.36 | 0.00 | 12.08 | | |
| **56** | - | - | 192.74 | 0.00 | 74.88 | 0.00 | | |
| **57** | - | - | 27.53 | 0.00 | 74.88 | 0.00 | | |
| **58** | - | - | - | - | - | - | | |
| **59** | - | - | - | - | - | - | | |
| **60** | - | - | - | - | - | - | | |
| **61** | - | - | - | - | - | - | | |
| **62** | - | - | - | - | - | - | | |
| **63** | - | - | - | - | - | - | | |
| **64** | - | - | - | - | - | - | | |
| **65** | - | - | - | - | - | - | | |
| **66** | - | - | - | - | - | - | | |
| **67** | - | - | - | - | - | - | | |
| **68** | - | - | - | - | - | - | | |
| **69** | - | - | - | - | - | - | | |
| **70** | - | - | - | - | - | - | | |
| **71** | - | - | - | - | - | - | | |
| **72** | - | - | - | - | - | - | | |
| **73** | - | - | - | - | - | - | | |
| **74** | - | - | - | - | - | - | | |
| **75** | - | - | - | - | - | - | | |
| **76** | - | - | - | - | - | - | | |
| **77** | - | - | - | - | - | - | | |
| **78** | - | - | - | - | - | - | | |
| **79** | - | - | - | - | - | - | | |
| **80** | - | - | - | - | - | - | | |
| **81** | - | - | - | - | - | - | | |
| **82** | - | - | - | - | - | - | | |
| **83** | - | - | - | - | - | - | | |
| **84** | - | - | - | - | - | - | | |
| **85** | - | - | - | - | - | - | | |
| **86** | - | - | - | - | - | - | | |
| **87** | - | - | - | - | - | - | | |
| **88** | - | - | - | - | - | - | | |
| **89** | - | - | - | - | - | - | | |
| **90** | - | - | - | - | - | - | | |
| **91** | - | - | - | - | - | - | | |
| **92** | - | - | - | - | - | - | | |
| **93** | - | - | - | - | - | - | | |
| **94** | - | - | - | - | - | - | | |
| **95** | - | - | - | - | - | - | | |
| **96** | - | - | - | - | - | - | | |
| **97** | - | - | - | - | - | - | | |
| **98** | - | - | - | - | - | - | | |
| **99** | - | - | - | - | - | - | | |
| **100** | - | - | - | - | - | - | | |
| **101** | - | - | - | - | - | - | | |
| **102** | - | - | - | - | - | - | | |
| **# of biomarkers per patient** | 10 | 10 | 17 | 11 | 14 | 12 | | |
| **% coverage** | 100.0% | 100.0% | 94.4% | 61.1% | 77.8% | 66.7% | | |

| **Seq ID** | **Grade 3 Huntington's Disease** | | | | | | | |
|---|---|---|---|---|---|---|---|---|
| - | SRR1759248 | SRR1759249 | SRR1759250 | SRR1759253 | SRR1759254 | SRR1759255 | SRR1759256 | SRR1759257 |
| **30** | - | - | - | - | - | - | - | - |
| **31** | - | - | - | - | - | - | - | - |
| **32** | - | - | - | - | - | - | - | - |
| **33** | - | - | - | - | - | - | - | - |
| **34** | - | - | - | - | - | - | - | - |
| **35** | - | - | - | - | - | - | - | - |
| **36** | - | - | - | - | - | - | - | - |
| **37** | - | - | - | - | - | - | - | - |
| **38** | - | - | - | - | - | - | - | - |
| **39** | - | - | - | - | - | - | - | - |
| **40** | - | - | - | - | - | - | - | - |
| **41** | - | - | - | - | - | - | - | - |
| **42** | - | - | - | - | - | - | - | - |
| **43** | - | - | - | - | - | - | - | - |
| **44** | - | - | - | - | - | - | - | - |
| **45** | - | - | - | - | - | - | - | - |
| **46** | - | - | - | - | - | - | - | - |
| **47** | - | - | - | - | - | - | - | - |
| **48** | - | - | - | - | - | - | - | - |
| **49** | - | - | - | - | - | - | - | - |
| **50** | - | - | - | - | - | - | - | - |
| **51** | - | - | - | - | - | - | - | - |
| **52** | - | - | - | - | - | - | - | - |
| **53** | - | - | - | - | - | - | - | - |
| **54** | - | - | - | - | - | - | - | - |
| **55** | - | - | - | - | - | - | - | - |
| **56** | - | - | - | - | - | - | - | - |
| **57** | - | - | - | - | - | - | - | - |
| **58** | 17.44 | 63.21 | 17.48 | 0.00 | 38.53 | 85.71 | 0.00 | 0.00 |
| **59** | 0.00 | 0.00 | 0.00 | 13.61 | 19.27 | 64.28 | 0.00 | 20.91 |
| **60** | 0.00 | 0.00 | 0.00 | 13.61 | 57.80 | 42.85 | 0.00 | 0.00 |
| **61** | 0.00 | 0.00 | 0.00 | 0.00 | 19.27 | 21.43 | 20.68 | 0.00 |
| **62** | 0.00 | 0.00 | 0.00 | 0.00 | 38.53 | 21.43 | 0.00 | 0.00 |
| **63** | 0.00 | 0.00 | 87.41 | 0.00 | 38.53 | 64.28 | 0.00 | 0.00 |
| **64** | 0.00 | 0.00 | 0.00 | 13.61 | 0.00 | 42.85 | 0.00 | 0.00 |
| **65** | 0.00 | 0.00 | 0.00 | 13.61 | 0.00 | 21.43 | 0.00 | 0.00 |
| **66** | 0.00 | 0.00 | 0.00 | 0.00 | 38.53 | 42.85 | 0.00 | 0.00 |
| **67** | 0.00 | 0.00 | 17.48 | 0.00 | 96.33 | 64.28 | 0.00 | 0.00 |
| **68** | 0.00 | 0.00 | 0.00 | 0.00 | 0.00 | 0.00 | 20.68 | 0.00 |
| **69** | 0.00 | 0.00 | 0.00 | 0.00 | 19.27 | 21.43 | 0.00 | 0.00 |
| **70** | 0.00 | 0.00 | 0.00 | 13.61 | 38.53 | 0.00 | 0.00 | 0.00 |
| **71** | 0.00 | 0.00 | 0.00 | 0.00 | 19.27 | 21.43 | 0.00 | 0.00 |
| **72** | 0.00 | 0.00 | 34.97 | 0.00 | 19.27 | 0.00 | 0.00 | 0.00 |
| **73** | 0.00 | 0.00 | 17.48 | 0.00 | 19.27 | 0.00 | 0.00 | 20.91 |
| **74** | 0.00 | 0.00 | 0.00 | 13.61 | 38.53 | 0.00 | 0.00 | 20.91 |
| **75** | 34.87 | 0.00 | 0.00 | 27.21 | 19.27 | 21.43 | 0.00 | 0.00 |
| **76** | 17.44 | 0.00 | 0.00 | 0.00 | 0.00 | 21.43 | 20.68 | 0.00 |
| **77** | 0.00 | 0.00 | 0.00 | 0.00 | 38.53 | 21.43 | 0.00 | 0.00 |
| **78** | 0.00 | 21.07 | 0.00 | 0.00 | 0.00 | 0.00 | 41.37 | 0.00 |
| **79** | 0.00 | 0.00 | 0.00 | 0.00 | 38.53 | 21.43 | 0.00 | 0.00 |
| **80** | 0.00 | 0.00 | 17.48 | 13.61 | 0.00 | 0.00 | 41.37 | 0.00 |
| **81** | 0.00 | 21.07 | 17.48 | 0.00 | 19.27 | 21.43 | 0.00 | 0.00 |
| **82** | 0.00 | 0.00 | 0.00 | 13.61 | 19.27 | 21.43 | 0.00 | 0.00 |
| **83** | 17.44 | 21.07 | 0.00 | 0.00 | 0.00 | 0.00 | 0.00 | 0.00 |
| **84** | 0.00 | 0.00 | 0.00 | 13.61 | 0.00 | 0.00 | 0.00 | 0.00 |
| **85** | 0.00 | 0.00 | 17.48 | 0.00 | 0.00 | 21.43 | 0.00 | 0.00 |
| **86** | - | - | - | - | - | - | - | - |
| **87** | - | - | - | - | - | - | - | - |
| **88** | - | - | - | - | - | - | - | - |
| **89** | - | - | - | - | - | - | - | - |
| **90** | - | - | - | - | - | - | - | - |
| **91** | - | - | - | - | - | - | - | - |
| **92** | - | - | - | - | - | - | - | - |
| **93** | - | - | - | - | - | - | - | - |
| **94** | - | - | - | - | - | - | - | - |
| **95** | - | - | - | - | - | - | - | - |
| **96** | - | - | - | - | - | - | - | - |
| **97** | - | - | - | - | - | - | - | - |
| **98** | - | - | - | - | - | - | - | - |
| **99** | - | - | - | - | - | - | - | - |
| **100** | - | - | - | - | - | - | - | - |
| **101** | - | - | - | - | - | - | - | - |
| **102** | - | - | - | - | - | - | - | - |
| **# of biomarkers per patient** | 4 | 4 | 8 | 10 | 19 | 19 | 5 | 3 |
| **% coverage** | 14.3% | 14.3% | 28.6% | 35.7% | 67.9% | 67.9% | 17.9% | 10.7% |
| - | SRR1759258 | SRR1759261 | SRR1759264 | SRR1759265 | SRR1759268 | SRR1759269 | SRR1759272 | |
| **30** | - | - | - | - | - | - | - | |
| **31** | - | - | - | - | - | - | - | |
| **32** | - | - | - | - | - | - | - | |
| **33** | - | - | - | - | - | - | - | |
| **34** | - | - | - | - | - | - | - | |
| **35** | - | - | - | - | - | - | - | |
| **36** | - | - | - | - | - | - | - | |
| **37** | - | - | - | - | - | - | - | |
| **38** | - | - | - | - | - | - | - | |
| **39** | - | - | - | - | - | - | - | |
| **40** | - | - | - | - | - | - | - | |
| **41** | - | - | - | - | - | - | - | |
| **42** | - | - | - | - | - | - | - | |
| **43** | - | - | - | - | - | - | - | |
| **44** | - | - | - | - | - | - | - | |
| **45** | - | - | - | - | - | - | - | |
| **46** | - | - | - | - | - | - | - | |
| **47** | - | - | - | - | - | - | - | |
| **48** | - | - | - | - | - | - | - | |
| **49** | - | - | - | - | - | - | - | |
| **50** | - | - | - | - | - | - | - | |
| **51** | - | - | - | - | - | - | - | |
| **52** | - | - | - | - | - | - | - | |
| **53** | - | - | - | - | - | - | - | |
| **54** | - | - | - | - | - | - | - | |
| **55** | - | - | - | - | - | - | - | |
| **56** | - | - | - | - | - | - | - | |
| **57** | - | - | - | - | - | - | - | |
| **58** | 0.00 | 0.00 | 0.00 | 0.00 | 0.00 | 17.02 | 17.44 | |
| **59** | 0.00 | 18.38 | 0.00 | 15.10 | 0.00 | 34.04 | 0.00 | |
| **60** | 0.00 | 36.76 | 0.00 | 15.10 | 0.00 | 34.04 | 0.00 | |
| **61** | 47.22 | 55.14 | 25.27 | 0.00 | 0.00 | 0.00 | 0.00 | |
| **62** | 0.00 | 110.27 | 0.00 | 15.10 | 0.00 | 85.10 | 0.00 | |
| **63** | 0.00 | 0.00 | 0.00 | 0.00 | 0.00 | 34.04 | 34.88 | |
| **64** | 0.00 | 91.89 | 0.00 | 15.10 | 0.00 | 85.10 | 0.00 | |
| **65** | 0.00 | 110.27 | 0.00 | 15.10 | 0.00 | 68.08 | 0.00 | |
| **66** | 0.00 | 36.76 | 0.00 | 0.00 | 0.00 | 85.10 | 17.44 | |
| **67** | 0.00 | 18.38 | 0.00 | 0.00 | 0.00 | 17.02 | 0.00 | |
| **68** | 0.00 | 110.27 | 12.64 | 30.20 | 0.00 | 34.04 | 0.00 | |
| **69** | 0.00 | 36.76 | 0.00 | 15.10 | 0.00 | 51.06 | 0.00 | |
| **70** | 0.00 | 18.38 | 0.00 | 15.10 | 0.00 | 51.06 | 0.00 | |
| **71** | 0.00 | 55.14 | 0.00 | 15.10 | 0.00 | 17.02 | 0.00 | |
| **72** | 0.00 | 0.00 | 12.64 | 0.00 | 0.00 | 17.02 | 34.88 | |
| **73** | 23.61 | 0.00 | 0.00 | 0.00 | 36.64 | 0.00 | 0.00 | |
| **74** | 0.00 | 18.38 | 25.27 | 0.00 | 0.00 | 0.00 | 0.00 | |
| **75** | 0.00 | 0.00 | 12.64 | 0.00 | 0.00 | 0.00 | 0.00 | |
| **76** | 0.00 | 36.76 | 0.00 | 0.00 | 0.00 | 17.02 | 0.00 | |
| **77** | 0.00 | 0.00 | 0.00 | 0.00 | 18.32 | 17.02 | 17.44 | |
| **78** | 0.00 | 18.38 | 12.64 | 0.00 | 0.00 | 17.02 | 0.00 | |
| **79** | 0.00 | 18.38 | 0.00 | 15.10 | 0.00 | 17.02 | 0.00 | |
| **80** | 0.00 | 18.38 | 0.00 | 0.00 | 18.32 | 0.00 | 0.00 | |
| **81** | 0.00 | 0.00 | 0.00 | 0.00 | 0.00 | 0.00 | 17.44 | |
| **82** | 23.61 | 0.00 | 0.00 | 0.00 | 0.00 | 17.02 | 0.00 | |
| **83** | 23.61 | 0.00 | 12.64 | 15.10 | 0.00 | 0.00 | 0.00 | |
| **84** | 0.00 | 18.38 | 0.00 | 0.00 | 18.32 | 17.02 | 17.44 | |
| **85** | 0.00 | 0.00 | 12.64 | 15.10 | 0.00 | 17.02 | 0.00 | |
| **86** | - | - | - | - | - | - | - | |
| **87** | - | - | - | - | - | - | - | |
| **88** | - | - | - | - | - | - | - | |
| **89** | - | - | - | - | - | - | - | |
| **90** | - | - | - | - | - | - | - | |
| **91** | - | - | - | - | - | - | - | |
| **92** | - | - | - | - | - | - | - | |
| **93** | - | - | - | - | - | - | - | |
| **94** | - | - | - | - | - | - | - | |
| **95** | - | - | - | - | - | - | - | |
| **96** | - | - | - | - | - | - | - | |
| **97** | - | - | - | - | - | - | - | |
| **98** | - | - | - | - | - | - | - | |
| **99** | - | - | - | - | - | - | - | |
| **100** | - | - | - | - | - | - | - | |
| **101** | - | - | - | - | - | - | - | |
| **102** | - | - | - | - | - | - | - | |
| **# of biomarkers per patient** | 4 | 18 | 8 | 12 | 4 | 21 | 7 | |
| **% coverage** | 14.3% | 64.3% | 28.6% | 42.9% | 14.3% | 75.0% | 25.0% | |

| **Seq ID** | **Grade 4 Huntington's Disease** | | | | | | | |
|---|---|---|---|---|---|---|---|---|
| **-** | SRR1759251 | SRR1759252 | SRR1759259 | SRR1759263 | SRR1759267 | SRR1759271 | SRR1759273 | |
| **30** | - | - | - | - | - | - | - | |
| **31** | - | - | - | - | - | - | - | |
| **32** | - | - | - | - | - | - | - | |
| **33** | - | - | - | - | - | - | - | |
| **34** | - | - | - | - | - | - | - | |
| **35** | - | - | - | - | - | - | - | |
| **36** | - | - | - | - | - | - | - | |
| **37** | - | - | - | - | - | - | - | |
| **38** | - | - | - | - | - | - | - | |
| **39** | - | - | - | - | - | - | - | |
| **40** | - | - | - | - | - | - | - | |
| **41** | - | - | - | - | - | - | - | |
| **42** | - | - | - | - | - | - | - | |
| **43** | - | - | - | - | - | - | - | |
| **44** | - | - | - | - | - | - | - | |
| **45** | - | - | - | - | - | - | - | |
| **46** | - | - | - | - | - | - | - | |
| **47** | - | - | - | - | - | - | - | |
| **48** | - | - | - | - | - | - | - | |
| **49** | - | - | - | - | - | - | - | |
| **50** | - | - | - | - | - | - | - | |
| **51** | - | - | - | - | - | - | - | |
| **52** | - | - | - | - | - | - | - | |
| **53** | - | - | - | - | - | - | - | |
| **54** | - | - | - | - | - | - | - | |
| **55** | - | - | - | - | - | - | - | |
| **56** | - | - | - | - | - | - | - | |
| **57** | - | - | - | - | - | - | - | |
| **58** | - | - | - | - | - | - | - | |
| **59** | - | - | - | - | - | - | - | |
| **60** | - | - | - | - | - | - | - | |
| **61** | - | - | - | - | - | - | - | |
| **62** | - | - | - | - | - | - | - | |
| **63** | - | - | - | - | - | - | - | |
| **64** | - | - | - | - | - | - | - | |
| **65** | - | - | - | - | - | - | - | |
| **66** | - | - | - | - | - | - | - | |
| **67** | - | - | - | - | - | - | - | |
| **68** | - | - | - | - | - | - | - | |
| **69** | - | - | - | - | - | - | - | |
| **70** | - | - | - | - | - | - | - | |
| **71** | - | - | - | - | - | - | - | |
| **72** | - | - | - | - | - | - | - | |
| **73** | - | - | - | - | - | - | - | |
| **74** | - | - | - | - | - | - | - | |
| **75** | - | - | - | - | - | - | - | |
| **76** | - | - | - | - | - | - | - | |
| **77** | - | - | - | - | - | - | - | |
| **78** | - | - | - | - | - | - | - | |
| **79** | - | - | - | - | - | - | - | |
| **80** | - | - | - | - | - | - | - | |
| **81** | - | - | - | - | - | - | - | |
| **82** | - | - | - | - | - | - | - | |
| **83** | - | - | - | - | - | - | - | |
| **84** | - | - | - | - | - | - | - | |
| **85** | - | - | - | - | - | - | - | |
| **86** | 20.28 | 0.00 | 0.00 | 41.85 | 0.00 | 29.21 | 18.83 | |
| **87** | 0.00 | 0.00 | 0.00 | 20.93 | 15.08 | 43.81 | 18.83 | |
| **88** | 0.00 | 0.00 | 0.00 | 20.93 | 15.08 | 14.60 | 18.83 | |
| **89** | 0.00 | 0.00 | 0.00 | 20.93 | 15.08 | 14.60 | 18.83 | |
| **90** | 0.00 | 14.55 | 14.29 | 0.00 | 0.00 | 14.60 | 18.83 | |
| **91** | 0.00 | 0.00 | 0.00 | 41.85 | 60.31 | 43.81 | 0.00 | |
| **92** | 40.55 | 0.00 | 0.00 | 0.00 | 0.00 | 14.60 | 18.83 | |
| **93** | 0.00 | 0.00 | 0.00 | 20.93 | 15.08 | 0.00 | 37.66 | |
| **94** | 0.00 | 14.55 | 42.86 | 0.00 | 0.00 | 14.60 | 0.00 | |
| **95** | 0.00 | 14.55 | 0.00 | 41.85 | 0.00 | 14.60 | 0.00 | |
| **96** | 0.00 | 29.09 | 0.00 | 20.93 | 0.00 | 0.00 | 18.83 | |
| **97** | 20.28 | 14.55 | 28.57 | 0.00 | 0.00 | 0.00 | 0.00 | |
| **98** | 20.28 | 0.00 | 0.00 | 20.93 | 15.08 | 0.00 | 0.00 | |
| **99** | 20.28 | 14.55 | 0.00 | 20.93 | 0.00 | 0.00 | 0.00 | |
| **100** | 0.00 | 0.00 | 14.29 | 20.93 | 0.00 | 0.00 | 18.83 | |
| **101** | 0.00 | 14.55 | 0.00 | 20.93 | 15.08 | 0.00 | 0.00 | |
| **102** | 0.00 | 14.55 | 0.00 | 20.93 | 15.08 | 0.00 | 0.00 | |
| **# of biomarkers per patient** | 5 | 8 | 4 | 13 | 8 | 9 | 9 | |
| **% coverage** | 29.4% | 47.1% | 23.5% | 76.5% | 47.1% | 52.9% | 52.9% | |

**Table 4A. Prognostic Specific Biomarkers for Huntington's Disease (<10years Disease Duration)**

| **Seq ID** | **sRNA Type** | **Sequence** | **Total Read Count** | **Frequency (Sensitivity)** | **Specificity** | **p-value in Discovery Set** |
|---|---|---|---|---|---|---|
| **122** | novel sRNA | ACAAGGTTCCGGCTGAGGAC | 71.66 | 60.00% | 100% | 7.71E-05 |
| **123** | IncRNA | CCGCGGGACGCCGCGGTGTCGTCCGCCGTCGCGCGGG | 63.56 | 60.00% | 100% | 7.71E-05 |
| **124** | novel sRNA | TGGCACACAGGACACGGACC | 63.56 | 60.00% | 100% | 7.71E-05 |
| **48** | novel sRNA | CGAGCTGACACTTTCCTTGGCATAGAGAACTTGGAGTA | 53.98 | 60.00% | 100% | 7.71E-05 |
| **55** | iso-miR-219a | GATGTCCAGCCACAATTCTC | 40.21 | 60.00% | 100% | 7.71E-05 |
| **125** | novel sRNA | GCTAGAGCCTGATGGAGCCTTGGACCGA | 40.21 | 60.00% | 100% | 7.71E-05 |
| **53** | novel sRNA | AGCACCTGACCCCGAGGACTGG | 40.21 | 60.00% | 100% | 7.71E-05 |
| **126** | novel sRNA | CATTTGGAGTGAACAGCCCGGA | 50.59 | 60.00% | 100% | 7.71E-05 |
| **127** | novel sRNA | TAAAGGTGGACTGACATTCCCTCT | 47.51 | 60.00% | 100% | 7.71E-05 |
| **128** | novel sRNA | ACAGAGTGGTAGAATCGGTAAGAACTCTGATT | 47.51 | 60.00% | 100% | 7.71E-05 |
| **129** | novel sRNA | AGCATGATTCGAAAGGAAACAAAATCGCCTGGAA | 40.21 | 60.00% | 100% | 7.71E-05 |
| **54** | novel sRNA | CTGCACCCCCTTCTTGGCTGT | 40.21 | 60.00% | 100% | 7.71E-05 |
| **130** | SNORA80E | TACCTGTGGGCTGTGAGCACTGAAGGGGGTTGCACAGTGAA | 49.2 | 60.00% | 100% | 7.71E-05 |
| **131** | novel sRNA | GCCCCCGAGCGCATCCTGGACCGCTGCTCCACCA | 40.21 | 60.00% | 100% | 7.71E-05 |
| **132** | iso-miR-30c | CGTTCCCGTGGTGTAAACATCCTACACTCTCAGCG | 40.21 | 60.00% | 100% | 7.71E-05 |
| **133** | novel sRNA | TTGGGTCTGTAGCACCTTGCATAGTGCC | 77.02 | 40.00% | 100% | 2.34E-03 |
| **134** | novel sRNA | CAATCATGGACCTTGTGCAGTTTTTTGTCACC | 48.65 | 40.00% | 100% | 2.34E-03 |
| **135** | iso-miR-504 | TGAAGGGAGTGCAGGGCAGGG | 59.58 | 40.00% | 100% | 2.34E-03 |
| **136** | novel sRNA | TGATTGGACTGAGGTGATCAGC | 59.58 | 40.00% | 100% | 2.34E-03 |

| **No.** | **SRR1759249** | **SRR1759262** | **SRR1759272** | **SRR1759270** | **SRR1759260** | |
|---|---|---|---|---|---|---|
| **122** | 42.14 | 0 | 17.44 | 12.08 | 0 | |
| **123** | 21.07 | 28.72 | 0 | 0 | 13.77 | |
| **124** | 21.07 | 28.72 | 0 | 0 | 13.77 | |
| **48** | 0 | 14.36 | 0 | 12.08 | 27.54 | |
| **55** | 0 | 14.36 | 0 | 12.08 | 13.77 | |
| **125** | 0 | 14.36 | 0 | 12.08 | 13.77 | |
| **53** | 0 | 14.36 | 0 | 12.08 | 13.77 | |
| **126** | 21.07 | 0 | 17.44 | 12.08 | 0 | |
| **127** | 21.07 | 14.36 | 0 | 12.08 | 0 | |
| **128** | 21.07 | 14.36 | 0 | 12.08 | 0 | |
| **129** | 0 | 14.36 | 0 | 12.08 | 13.77 | |
| **54** | 0 | 14.36 | 0 | 12.08 | 13.77 | |
| **130** | 21.07 | 14.36 | 0 | 0 | 13.77 | |
| **131** | 0 | 14.36 | 0 | 12.08 | 13.77 | |
| **132** | 0 | 14.36 | 0 | 12.08 | 13.77 | |
| **133** | 42.14 | 0 | 34.88 | 0 | 0 | |
| **134** | 0 | 0 | 34.88 | 0 | 13.77 | |
| **135** | 42.14 | 0 | 17.44 | 0 | 0 | |
| **136** | 42.14 | 0 | 17.44 | 0 | 0 | |
| **# of biomarkers per patient** | 10 | 13 | 6 | 12 | 12 | |
| **% coverage** | 50.0% | 65.0% | 30.0% | 60.0% | 60.0% | |

**Table 4B. Prognostic Specific Biomarkers for Huntington's Disease (>20years Disease Duration)**

| **Seq ID** | **sRNA Type** | **Sequence** | **Total Read Count** | **Frequency (Sensitivity)** | **Specificity** | **p-value in Discovery Set** | | |
|---|---|---|---|---|---|---|---|---|
| **1** | iso-miR-10b | AACCCTGTAGAACCGAATTTGTG | 385.34 | 56.25% | 100% | 2.00E-08 | | |
| **2** | iso-miR-10b | CACCCTGTAGAACCGAATTTGTG | 315.56 | 56.25% | 100% | 2.00E-08 | | |
| **12** | iso-miR-10b | ACCCTGTAGAACTGAATTTGTGT | 293.64 | 50.00% | 100% | 2.00E-07 | | |
| **7** | iso-miR-10b | TACCCTGTAGAATCGAATTTG | 257.77 | 50.00% | 100% | 2.00E-07 | | |
| **11** | iso-miR-196a | TAG GTAGTTTCATGTTGTTGG GAT | 192.47 | 50.00% | 100% | 2.00E-07 | | |
| **19** | iso-miR-10b | TACCCTGTCGAACCGAATTTGT | 808.19 | 43.75% | 100% | 1.80E-06 | | |
| **13** | iso-miR-10b | TACCCTGTAGAACCGAATTTGAG | 214.05 | 43.75% | 100% | 1.80E-06 | | |
| **103** | iso-miR-10b | ACCCTGTAGAACCGAATTTGTT | 208.46 | 43.75% | 100% | 1.80E-06 | | |
| **104** | novel sRNA | CACCTGTGAACTCAAAAGGCTCTTTT CAGCGCCCCT | 182.5 | 43.75% | 100% | 1.80E-06 | | |
| **105** | novel sRNA | TCATGACCCCATGTCTAACAACATGGCTA | 167.96 | 43.75% | 100% | 1.80E-06 | | |
| **106** | iso-miR-101-2 | AACAGTACTGTGATAACTGAAGT | 160.89 | 43.75% | 100% | 1.80E-06 | | |
| **107** | iso-miR-99b | CAGTTCTACAGTCCGACGATCCACCCG TAGAACCGACCTTGC | 173.73 | 43.75% | 100% | 1.80E-06 | | |
| **108** | iso-miR-10b | ACCCTGTAGAACCGAATTGGTG | 158.84 | 43.75% | 100% | 1.80E-06 | | |
| **15** | novel sRNA | ATTACTCCTGCCATCATGACCCTTGGCCATAAT | 167.14 | 43.75% | 100% | 1.80E-06 | | |
| **109** | iso-miR-10b | ACCCTGTAGAACCAAATTTGTGA | 135.45 | 43.75% | 100% | 1.80E-06 | | |
| **18** | iso-miR-10b | ACACTGTAGAACCGAATTTGTG | 123.55 | 43.75% | 100% | 1.80E-06 | | |
| **24** | iso-miR-10b | ACCCTGTAGAACCGAATTTATG | 390.01 | 37.50% | 100% | 1.48E-05 | | |
| **20** | iso-miR-10b | TACCCTGTAGCACCGAATTTGTGA | 400.57 | 37.50% | 100% | 1.48E-05 | | |
| **110** | iso-miR-10b | AGCCTGTAGAACCGAATTTGTGA | 228.85 | 37.50% | 100% | 1.48E-05 | | |
| **111** | iso-miR-10b | ACCCTGTAGAACCGAATTTATGA | 186.64 | 37.50% | 100% | 1.48E-05 | | |
| **61** | SCARNA10 | CCACATGATGATACCAAGGCTGTTG | 189.02 | 37.50% | 100% | 1.48E-05 | | |
| **112** | iso-miR-10b | TACCTTGTAGAACCGAATTTGTG | 149.15 | 37.50% | 100% | 1.48E-05 | | |
| **113** | iso-miR-10b | CACCCTGTAGAACCGAATTTGTGA | 175.33 | 37.50% | 100% | 1.48E-05 | | |
| **114** | iso-miR-10b | ACCCTGTAGAACCGAAGTTGTG | 205.61 | 37.50% | 100% | 1.48E-05 | | |
| **115** | iso-miR-10b | ACCCTGTAGAACCGAATTTCTG | 141 | 37.50% | 100% | 1.48E-05 | | |
| **116** | novel sRNA | CTCCCTGATGATTCTGAAATACACTACTGAAC | 119.52 | 37.50% | 100% | 1.48E-05 | | |
| **117** | iso-miR-10b | GCCCTGTAGAACCGAATTTGTGT | 132.7 | 37.50% | 100% | 1.48E-05 | | |
| **118** | novel sRNA | TTTGTAGGACTCAGCCAGACG | 116.15 | 37.50% | 100% | 1.48E-05 | | |
| **119** | novel sRNA | ATCATCATCCTAGCCCTAAGTCTGGC | 112.62 | 37.50% | 100% | 1.48E-05 | | |
| **120** | iso-miR-10b | ACCCTGGAGAACCGAATTTGTG | 112.03 | 37.50% | 100% | 1.48E-05 | | |
| **121** | tRNA-derived sRNA | TGTAATGGTTAGCACTCTGGACTCTGAATCCATT | 111.94 | 37.50% | 100% | 1.48E-05 | | |

| **Seq ID** | **SRR1759269** | **SRR1759255** | **SRR1759248** | **SRR1759264** | **SRR1759261** | **SRR1759258** | **SRR1759259** | **SRR1759273** |
|---|---|---|---|---|---|---|---|---|
| **1** | 0 | 42.86 | 0 | 0 | 0 | 47.22 | 57.16 | 18.83 |
| **2** | 34.04 | 0 | 0 | 0 | 0 | 0 | 85.74 | 56.49 |
| **12** | 17.02 | 0 | 0 | 25.28 | 0 | 47.22 | 14.29 | 0 |
| **7** | 0 | 0 | 17.44 | 25.28 | 0 | 0 | 28.58 | 0 |
| **11** | 0 | 21.43 | 17.44 | 12.64 | 0 | 0 | 14.29 | 18.83 |
| **19** | 0 | 0 | 17.44 | 0 | 0 | 23.61 | 0 | 0 |
| **13** | 17.02 | 0 | 17.44 | 63.4 | 0 | 0 | 14.29 | 18.83 |
| **103** | 0 | 64.29 | 0 | 0 | 0 | 0 | 14.29 | 37.66 |
| **104** | 34.04 | 21.43 | 0 | 0 | 18.38 | 0 | 0 | 37.66 |
| **105** | 17.02 | 0 | 0 | 0 | 18.38 | 0 | 14.29 | 37.66 |
| **106** | 0 | 21.43 | 34.87 | 12.64 | 0 | 23.61 | 28.58 | 18.83 |
| **107** | 0 | 21.43 | 34.87 | 0 | 0 | 23.61 | 0 | 37.66 |
| **108** | 0 | 0 | 0 | 12.64 | 0 | 23.61 | 28.58 | 18.83 |
| **15** | 17.02 | 21.43 | 17.44 | 0 | 0 | 47.22 | 0 | 0 |
| **109** | 0 | 0 | 0 | 12.64 | 0 | 0 | 14.29 | 18.83 |
| **18** | 0 | 21.43 | 0 | 0 | 18.38 | 0 | 0 | 18.83 |
| **24** | 17.02 | 0 | 0 | 0 | 18.38 | 0 | 0 | 75.32 |
| **20** | 0 | 0 | 0 | 0 | 0 | 23.61 | 157.19 | 0 |
| **110** | 0 | 21.43 | 0 | 25.28 | 0 | 0 | 57.16 | 18.83 |
| **111** | 17.02 | 0 | 0 | 0 | 0 | 0 | 0 | 0 |
| **61** | 0 | 21.43 | 0 | 25.28 | 55.14 | 47.22 | 0 | 0 |
| **112** | 0 | 0 | 0 | 37.92 | 0 | 23.61 | 0 | 37.66 |
| **113** | 0 | 21.43 | 0 | 0 | 0 | 23.61 | 0 | 0 |
| **114** | 0 | 0 | 17.44 | 12.64 | 0 | 0 | 0 | 0 |
| **115** | 0 | 0 | 0 | 0 | 18.38 | 0 | 0 | 37.66 |
| **116** | 0 | 0 | 17.44 | 12.64 | 0 | 0 | 0 | 0 |
| **117** | 0 | 21.43 | 0 | 0 | 0 | 0 | 14.29 | 18.83 |
| **118** | 17.02 | 0 | 17.44 | 0 | 0 | 23.61 | 0 | 18.83 |
| **119** | 17.02 | 21.43 | 0 | 0 | 0 | 23.61 | 0 | 0 |
| **120** | 17.02 | 21.43 | 0 | 0 | 0 | 0 | 0 | 18.83 |
| **121** | 0 | 21.43 | 17.44 | 0 | 0 | 0 | 14.29 | 18.83 |
| **# of biomarkers per patient** | 12 | 16 | 11 | 13 | 6 | 13 | 15 | 21 |
| **% coverage** | 38.7% | 51.6% | 35.5% | 41.9% | 19.4% | 41.9% | 48.4% | 67.7% |

| **No.** | **SRR1759267** | **SRR1759263** | **SRR1759256** | **SRR1759252** | **SRR1759268** | **SRR1759254** | **SRR1759271** | **SRR1759251** |
|---|---|---|---|---|---|---|---|---|
| **1** | 0 | 0 | 41.36 | 43.65 | 0 | 38.54 | 14.6 | 81.12 |
| **2** | 15.08 | 0 | 20.68 | 29.1 | 0 | 19.27 | 14.6 | 40.56 |
| **12** | 75.4 | 20.93 | 0 | 0 | 54.96 | 38.54 | 0 | 0 |
| **7** | 75.4 | 0 | 0 | 0 | 36.64 | 19.27 | 14.6 | 40.56 |
| **11** | 0 | 20.93 | 0 | 29.1 | 0 | 57.81 | 0 | 0 |
| **19** | 316.68 | 230.23 | 0 | 0 | 54.96 | 19.27 | 146 | 0 |
| **13** | 0 | 62.79 | 0 | 0 | 0 | 0 | 0 | 20.28 |
| **103** | 15.08 | 0 | 0 | 0 | 18.32 | 38.54 | 0 | 20.28 |
| **104** | 15.08 | 0 | 0 | 0 | 36.64 | 19.27 | 0 | 0 |
| **105** | 0 | 20.93 | 41.36 | 0 | 18.32 | 0 | 0 | 0 |
| **106** | 0 | 20.93 | 0 | 0 | 0 | 0 | 0 | 0 |
| **107** | 0 | 20.93 | 20.68 | 14.55 | 0 | 0 | 0 | 0 |
| **108** | 0 | 0 | 41.36 | 14.55 | 0 | 19.27 | 0 | 0 |
| **15** | 0 | 0 | 0 | 14.55 | 0 | 0 | 29.2 | 20.28 |
| **109** | 30.16 | 20.93 | 0 | 0 | 18.32 | 0 | 0 | 20.28 |
| **18** | 15.08 | 0 | 20.68 | 14.55 | 0 | 0 | 14.6 | 0 |
| **24** | 120.64 | 41.85 | 0 | 0 | 0 | 0 | 116.8 | 0 |
| **20** | 15.08 | 20.93 | 165.44 | 0 | 18.32 | 0 | 0 | 0 |
| **110** | 0 | 0 | 0 | 14.55 | 91.6 | 0 | 0 | 0 |
| **111** | 30.16 | 62.79 | 0 | 14.55 | 18.32 | 0 | 43.8 | 0 |
| **61** | 0 | 0 | 20.68 | 0 | 0 | 19.27 | 0 | 0 |
| **112** | 15.08 | 0 | 0 | 0 | 0 | 0 | 14.6 | 20.28 |
| **113** | 0 | 0 | 0 | 14.55 | 36.64 | 38.54 | 0 | 40.56 |
| **114** | 0 | 20.93 | 0 | 0 | 18.32 | 0 | 14.6 | 121.68 |
| **115** | 30.16 | 20.93 | 0 | 0 | 0 | 19.27 | 14.6 | 0 |
| **116** | 30.16 | 0 | 20.68 | 0 | 18.32 | 0 | 0 | 20.28 |
| **117** | 0 | 0 | 0 | 0 | 18.32 | 19.27 | 0 | 40.56 |
| **118** | 0 | 20.93 | 0 | 0 | 18.32 | 0 | 0 | 0 |
| **119** | 15.08 | 20.93 | 0 | 14.55 | 0 | 0 | 0 | 0 |
| **120** | 0 | 20.93 | 0 | 14.55 | 0 | 19.27 | 0 | 0 |
| **121** | 0 | 0 | 20.68 | 0 | 0 | 19.27 | 0 | 0 |
| **# of biomarkers per patient** | 15 | 16 | 10 | 12 | 15 | 16 | 11 | 13 |
| **% coverage** | 48.4% | 51.6% | 32.3% | 38.7% | 48.4% | 51.6% | 35.5% | 41.9% |

**Table 5. 18 Biomarker Panel**

| **HDB** | **SEQ ID NO:** | **Biomarker Sequence (shown as DNA)** |
|---|---|---|
| **1** | **1** | AACCCTGTAGAACCGAATTTGTG |
| **2** | **2** | CACCCTGTAGAACCGAATTTGTG |
| **3** | **3** | TAGGTAGTTTCATGTTGTTGGGAA |
| **4** | **6** | TAGGTAGTTTCATGTTGTTGGGT |
| **5** | **7** | TACCCTGTAGAATCGAATTTG |
| **6** | **9** | GCCCTGTAGAACCGAATTTGT |
| **7** | **10** | TACCCTGTAGAACCGAATTTGTGTGG |
| **8** | **11** | TAGGTAGTTTCATGTTGTTGGGAT |
| **9** | **12** | ACCCTGTAGAACTGAATTTGTGT |
| **10** | **22** | CCCGTGGACAAGTCAGGCTCTTGGGACCTT |
| **11** | **23** | TCAGTGCACTACAGAACTTTTA |
| **12** | **25** | ACCCTGTAGAACCGAGTTTGTG |
| **13** | **26** | TTAAAGCACGTGTTAGACTG |
| **14** | **27** | TACCCATTGCATATCGGAATTGT |
| **15** | **55** | GATGTCCAGCCACAATTCTC |
| **16** | **40** | GTCTCTGTGGCGCAATCGGTTAGCGCTTCGGCT |
| **17** | **41** | CTGAGGCTGCAGGATCGCTTGAGTCCAGGAG |
| **18** | **137** | AGTAAGGTAAGCTAAATAAGCTATCGGGACCACCA |

**TABLE 6. Primers and probes used for RT-qPCR analysis of binary small RNA classifiers.**

| | **RT Primer (5' to 3')** | **Forward Primer (5' to 3')** | **Reverse Primer (5' to 3')** | **TaqMan Probe (5' to 3')** |
|---|---|---|---|---|
| **HDB-1** | GTCGTATCCAGTGCAGGGTCCGAGGTATTCGCACTGGATACGACcacaaa (SEQ ID NO:138) | TGCGGAACCCTGTAGAACCG (SEQ ID NO:139) | TGGAGCCTGGGACGTG (SEQ ID NO:140) | TACGACCACAAATTC (SEQ ID NO:141) |
| **HDB-2** | GTCGTATCCAGTGCAGGGTCCGAGGTATTCGCACTGGATACGACcacaaa (SEQ ID NO:138) | TGCGGCACCCTGTAGAACCG (SEQ ID NO:142) | TGGAGCCTGGGACGTG (SEQ ID NO:140) | TACGACCACAAATTC (SEQ ID NO:141) |
| **HDB-3** | GTCGTATCCAGTGCAGGGTCCGAGGTATTCGCACTGGATACGACttccca (SEQ ID NO:143) | TGCGGTAGGTAGTTTCATGTTG (SEQ ID NO:144) | TGGAGCCTGGGACGTG (SEQ ID NO:140) | TACGACTTCCCAACA (SEQ ID NO:145) |
| **HDB-4** | GTCGTATCCAGTGCAGGGTCCGAGGTATTCGCACTGGATACGACacccaa (SEQ ID NO:146) | TGCGGTAGGTAGTTTCATGTTG (SEQ ID NO:144) | TGGAGCCTGGGACGTG (SEQ ID NO:140) | TACGACACCCAACAA (SEQ ID NO:147) |
| **HDB-5** | GTCGTATCCAGTGCAGGGTCCGAGGTATTCGCACTGGATACGACcaaatt (SEQ ID NO:148) | TGCGGTACCCTGTAGAATC (SEQ ID NO:149) | TGGAGCCTGGGACGTG (SEQ ID NO:140) | TACGACCAAATTCGA (SEQ ID NO:150) |
| **HDB-6** | GTCGTATCCAGTGCAGGGTCCGAGGTATTCGCACTGGATACGACacaaat (SEQ ID NO:151) | TGCGGGCCCTGTAGAACC (SEQ ID NO:152) | TGGAGCCTGGGACGTG (SEQ ID NO:140) | TACGACACAAATTCG (SEQ ID NO:153) |
| **HDB-7** | GTCGTATCCAGTGCAGGGTCCGAGGTATTCGCACTGGATACGACccacac (SEQ ID NO:154) | TGCGGTACCCTGTAGAACCGAAT (SEQ ID NO:155) | TGGAGCCTGGGACGTG (SEQ ID NO:140) | TACGACGGTGTGTTT (SEQ ID NO:156) |
| **HDB-8** | GTCGTATCCAGTGCAGGGTCCGAGGTATTCGCACTGGATACGACatccca (SEQ ID NO:157) | TGCGGTAGGTAGTTTCATGTTG (SEQ ID NO:144) | TGGAGCCTGGGACGTG (SEQ ID NO:140) | TACGACTAGGGTTGT (SEQ ID NO:158) |
| **HDB-9** | GTCGTATCCAGTGCAGGGTCCGAGGTATTCGCACTGGATACGACacacaa (SEQ ID NO:159) | TGCGGACCCTGTAGAACTG (SEQ ID NO:160) | TGGAGCCTGGGACGTG (SEQ ID NO:140) | TACGACTGTGTTTAA (SEQ ID NO:161) |
| **HDB-10** | GTCGTATCCAGTGCAGGGTCCGAGGTATTCGCACTGGATACGACaaggtc (SEQ ID NO:162) | TGCGGCCCGTGGACAAGTCAGGCTCTTG (SEQ ID NO:163) | TGGAGCCTGGGACGTG (SEQ ID NO:140) | TACGACTTCCAGGGT (SEQ ID NO:164) |
| **HDB-11** | GTCGTATCCAGTGCAGGGTCCGAGGTATTCGCACTGGATACGACtaaaag (SEQ ID NO:165) | TGCGGTCAGTGCACTACAG (SEQ ID NO:166) | TGGAGCCTGGGACGTG (SEQ ID NO:140) | TACGACATTTTCAAG (SEQ ID NO:167) |
| **HDB-12** | GTCGTATCCAGTGCAGGGTCCGAGGTATTCGCACTGGATACGACcacaaa (SEQ ID NO:138) | TGCGGACCCTGTAGAACCG (SEQ ID NO:139) | TGGAGCCTGGGACGTG (SEQ ID NO:140) | TACGACGTGTTTGAG (SEQ ID NO:168) |
| **HDB-13** | GTCGTATCCAGTGCAGGGTCCGAGGTATTCGCACTGGATACGACcagtct (SEQ ID NO:169) | TGCGGTTAAAGCACGTG (SEQ ID NO:170) | TGGAGCCTGGGACGTG (SEQ ID NO:140) | TACGACGTCACATTG (SEQ ID NO:171) |
| **HDB-14** | GTCGTATCCAGTGCAGGGTCCGAGGTATTCGCACTGGATACGACacaatt (SEQ ID NO:172) | TGCGGTACCCATTGCATATC (SEQ ID NO:173) | TGGAGCCTGGGACGTG (SEQ ID NO:140) | TACGACTGTTAAGGC (SEQ ID NO:174) |
| **HDB-15** | GTCGTATCCAGTGCAGGGTCCGAGGTATTCGCACTGGATACGACgagaat (SEQ ID NO:175) | TGCGGGATGTCCAGCCAC (SEQ ID NO:176) | TGGAGCCTGGGACGTG (SEQ ID NO:140) | TACGACCTCTTAACA (SEQ ID NO:177 |
| **HDB-16** | GTCGTATCCAGTGCAGGGTCCGAGGTATTCGCACTGGATACGACagccga (SEQ ID NO:178) | TGCGGGTCTCTGTGGCGCAATCGGTTAGCG (SEQ ID NO:179) | TGGAGCCTGGGACGTG (SEQ ID NO:140) | TACGACTCGGCTTCG (SEQ ID NO:180) |
| **HDB-17** | GTCGTATCCAGTGCAGGGTCCGAGGTATTCGCACTGGATACGACctcctg (SEQ ID NO:181) | TGCGGCTGAGGCTGCAGGATCGCTTGAG (SEQ ID NO:182) | TGGAGCCTGGGACGTG (SEQ ID NO:140) | TACGACGAGGACCTG (SEQ ID NO:183) |
| **HDB-18** | cTCCTATCCACTCCACCCTCCCACCTATTCCCACTCCATACCACtggtgg (SEQ ID NO:184) | TGCGGAGTAAGGTAAGCTAAATAAGCTATCGG (SEQ ID NO:185) | TGGAGCCTGGGACGTG (SEQ ID NO:140) | TACGACACCACCAGG (SEQ ID NO:186) |

## Claims

1. An *in vitro* method for evaluating Huntington's disease in a subject, the method comprising:
providing a biological sample from a subject having an expanded trinucleotide repeat in a Huntingtin gene, or providing RNA extracted therefrom,
determining the presence or absence of the sRNA predictors according to SEQ ID NO: 6 or SEQ ID NO: 1 in the sample, wherein the presence of the sRNA predictors is indicative of Huntington's disease activity, optionally wherein the relative or absolute amount of the sRNA predictors is determined.

2. The method of claim 1, wherein further the presence or absence of sRNA predictors according to SEQ ID NO: 7, SEQ ID NO: 10, SEQ ID NO: 2, SEQ ID NO: 3, SEQ ID NO: 9, SEQ ID NO: 11, SEQ ID NO: 12, SEQ ID NO: 22, SEQ ID NO: 23, SEQ ID NO: 25, SEQ ID NO: 26, SEQ ID NO: 27, SEQ ID NO: 55, SEQ ID NO: 40, SEQ ID NO: 41, SEQ ID NO: 137 are determined in the sample, wherein the presence of the sRNA predictors is indicative of Huntington's disease activity, optionally wherein the relative or absolute amount of the sRNA predictors is determined.

3. The method of claim 2, wherein the presence or absence of at least five or at least ten of the sRNAs are determined.

4. The method of any one of claims 1 to 3, wherein the sample is a biological fluid, wherein the biological fluid is selected from blood, serum, plasma, urine, saliva, and cerebrospinal fluid.

5. The method of any one of claims 1 to 3, wherein the sample is a solid tissue, which is optionally brain tissue.

6. The method of any one of claims 1 to 5, wherein the presence or absence of the sRNAs are determined by a quantitative or qualitative PCR assay.

7. The method of claim 6, wherein the presence or absence of sRNAs are determined using a fluorescent dye or fluorescent-labeled probe, optionally, wherein the probe further comprises a quencher moiety, optionally, wherein sRNAs are amplified using a stem-loop RT primer.

8. The method of any one of claims 1 to 5, wherein the presence or absence of sRNAs is determined using a hybridization assay.

9. The method of any one of claims 1 to 5, wherein the presence or absence of the sRNAs are determined by nucleic acid sequencing, and sRNAs are identified by a process that comprises trimming a 3' sequencing adaptor from individual sRNA sequences.

10. The method of any one of claims 1 to 9, wherein the subject has an HTT allele selected from a full penetrance allele, a reduced penetrance allele, and an intermediate penetrance allele.

11. The method of any one of claims 1 to 10, wherein the subject is asymptomatic.

12. The method of any one of claims 1 to 11, wherein the subject has Grade 1 HD, Grade 2 HD, Grade 3 HD, or Grade 4 HD.

13. The method of any one of claims 1 to 12, wherein the method is repeated, optionally wherein a subject is evaluated at a frequency of at least about once per year.

14. The method of any one of claims 1 to 13, wherein the subject is undergoing a therapy or candidate therapy for HD or HD symptoms.

15. Use of a kit for evaluating samples for Huntington's disease activity, comprising:
sRNA-specific probes and/or primers configured for performing the method of any one of claims 1 to 14, for detecting sRNAs according to SEQ ID NO: 6 or SEQ ID NO: 1.

16. The use of claim 15, further comprising sRNA-specific probes and/or primers for detecting sRNAs according to SEQ ID NO: 7, SEQ ID NO: 10, SEQ ID NO: 2, SEQ ID NO: 3, SEQ ID NO: 9, SEQ ID NO: 11, SEQ ID NO: 12, SEQ ID NO: 22, SEQ ID NO: 23, SEQ ID NO: 25, SEQ ID NO: 26, SEQ ID NO: 27, SEQ ID NO: 55, SEQ ID NO: 40, SEQ ID NO: 41, SEQ ID NO: 137.

## Patentansprüche

1. In-vitro-Verfahren zur Untersuchung der Huntington-Krankheit bei einem Subjekt, wobei das Verfahren umfasst:
Bereitstellen einer biologischen Probe eines Subjekts, das eine erweiterte Trinukleotidwiederholung in einem Huntingtin-Gen aufweist, oder Bereitstellen von daraus extrahierter RNA,
Bestimmen der Anwesenheit oder Abwesenheit der sRNA-Prädiktoren gemäß SEQ ID NO: 6 oder SEQ ID NO: 1 in der Probe, wobei die Anwesenheit der sRNA-Prädiktoren auf die Aktivität der Huntington-Krankheit hinweist, optional wobei die relative oder absolute Menge der sRNA-Prädiktoren bestimmt wird.

2. Verfahren nach Anspruch 1, wobei ferner die Anwesenheit oder Abwesenheit von sRNA-Prädiktoren gemäß SEQ ID NO: 7, SEQ ID NO: 10, SEQ ID NO: 2, SEQ ID NO: 3, SEQ ID NO: 9, SEQ ID NO: 11, SEQ ID NO: 12, SEQ ID NO: 22, SEQ ID NO: 23, SEQ ID NO: 25, SEQ ID NO: 26, SEQ ID NO: 27, SEQ ID NO: 55, SEQ ID NO: 40, SEQ ID NO: 41, SEQ ID NO: 137 in der Probe bestimmt wird, wobei die Anwesenheit der sRNA-Prädiktoren auf die Aktivität der Huntington-Krankheit hinweist, optional wobei die relative oder absolute Menge der sRNA-Prädiktoren bestimmt wird.

3. Verfahren nach Anspruch 2, wobei die Anwesenheit oder Abwesenheit von mindestens fünf oder mindestens zehn der sRNAs bestimmt wird.

4. Verfahren nach einem der Ansprüche 1 bis 3, wobei es sich bei der Probe um eine biologische Flüssigkeit handelt, wobei die biologische Flüssigkeit ausgewählt ist aus Blut, Serum, Plasma, Urin, Speichel und Zerebrospinalflüssigkeit.

5. Verfahren nach einem der Ansprüche 1 bis 3, wobei es sich bei der Probe um ein festes Gewebe handelt, bei dem es sich optional um Gehirngewebe handelt.

6. Verfahren nach einem der Ansprüche 1 bis 5, wobei die Anwesenheit oder Abwesenheit der sRNAs durch einen quantitativen oder qualitativen PCR-Assay bestimmt wird.

7. Verfahren nach Anspruch 6, wobei die Anwesenheit oder Abwesenheit von sRNAs unter Verwendung eines Fluoreszenzfarbstoffs oder einer fluoreszenzmarkierten Sonde bestimmt wird, optional wobei die Sonde ferner einen Quencheranteil umfasst, optional wobei die sRNAs unter Verwendung eines Stem-Loop-RT-Primers amplifiziert werden.

8. Verfahren nach einem der Ansprüche 1 bis 5, wobei die Anwesenheit oder Abwesenheit von sRNAs unter Verwendung eines Hybridisierungsassays bestimmt wird.

9. Verfahren nach einem der Ansprüche 1 bis 5, wobei die Anwesenheit oder Abwesenheit der sRNAs durch Nukleinsäuresequenzierung bestimmt wird und die sRNAs durch ein Verfahren identifiziert werden, das das Trimmen eines 3'-Sequenzierungsadaptors von einzelnen sRNA-Sequenzen umfasst.

10. Verfahren nach einem der Ansprüche 1 bis 9, wobei das Subjekt ein HTT-Allel aufweist, das aus einem Allel mit voller Penetranz, einem Allel mit reduzierter Penetranz und einem Allel mit intermediärer Penetranz ausgewählt ist.

11. Verfahren nach einem der Ansprüche 1 bis 10, wobei das Subjekt asymptomatisch ist.

12. Verfahren nach einem der Ansprüche 1 bis 11, wobei das Subjekt HD Grad 1, HD Grad 2, HD Grad 3 oder HD Grad 4 aufweist.

13. Verfahren nach einem der Ansprüche 1 bis 12, wobei das Verfahren wiederholt wird, optional wobei ein Subjekt mit einer Häufigkeit von mindestens etwa einmal pro Jahr untersucht wird.

14. Verfahren nach einem der Ansprüche 1 bis 13, wobei sich das Subjekt einer Therapie oder einer Therapie eines Kandidaten für HD oder HD-Symptome unterzieht.

15. Verwendung eines Kits zur Auswertung von Proben auf die Aktivität der Huntington-Krankheit, umfassend:
sRNA-spezifische Sonden und/oder Primer, die zur Durchführung des Verfahrens nach einem der Ansprüche 1 bis 14 konfiguriert sind, zum Nachweis von sRNAs gemäß SEQ ID NO: 6 oder SEQ ID NO: 1.

16. Verwendung nach Anspruch 15, ferner umfassend sRNA-spezifische Sonden und/oder Primer zum Nachweis von sRNAs gemäß SEQ ID NO: 7, SEQ ID NO: 10, SEQ ID NO: 2, SEQ ID NO: 3, SEQ ID NO: 9, SEQ ID NO: 11, SEQ ID NO: 12, SEQ ID NO: 22, SEQ ID NO: 23, SEQ ID NO: 25, SEQ ID NO: 26, SEQ ID NO: 27, SEQ ID NO: 55, SEQ ID NO: 40, SEQ ID NO: 41, SEQ ID NO: 137.

## Revendications

1. Procédé *in vitro* d'évaluation de la maladie de Huntington chez un sujet, le procédé comprenant :
la fourniture d'un échantillon biologique provenant d'un sujet présentant une répétition de trinucléotides étendue dans un gène de la huntingtine, ou la fourniture d'un ARN extrait de celui-ci,
la détermination de la présence ou de l'absence de prédicteurs d'ARNs selon SEQ ID NO : 6 ou SEQ ID NO : 1 dans l'échantillon, dans lequel la présence des prédicteurs d'ARNs indique une activité de la maladie de Huntington, éventuellement dans lequel la quantité relative ou absolue des prédicteurs d'ARNs est déterminée.

2. Procédé selon la revendication 1, dans lequel en outre la présence ou l'absence des prédicteurs d'ARNs selon SEQID NO : 7, SEQ ID NO : 10, SEQID NO : 2, SEQID NO : 3, SEQID NO : 9, SEQID NO : 11, SEQ ID NO : 12, SEQ ID NO : 22, SEQ ID NO : 23, SEQ ID NO : 25, SEQ ID NO : 26, SEQ ID NO : 27, SEQ ID NO : 55, SEQ ID NO : 40, SEQ ID NO : 41, SEQ ID NO : 137 est déterminée dans l'échantillon, dans lequel la présence des prédicteurs d'ARNs indique une activité de la maladie de Huntington, éventuellement dans lequel la quantité relative ou absolue des prédicteurs d'ARNs est déterminée.

3. Procédé selon la revendication 2, dans lequel la présence ou l'absence d'au moins cinq ou d'au moins dix des ARNs est déterminée.

4. Procédé selon l'une quelconque des revendications 1 à 3, dans lequel l'échantillon est un fluide biologique, dans lequel le fluide biologique est sélectionné parmi du sang, du sérum, du plasma, de l'urine, de la salive et du fluide céphalorachidien.

5. Procédé selon l'une quelconque des revendications 1 à 3, dans lequel l'échantillon est un tissu solide, qui est éventuellement du tissu cérébral.

6. Procédé selon l'une quelconque des revendications 1 à 5, dans lequel la présence ou l'absence des ARNs est déterminée par un dosage par PCR quantitatif ou qualitatif.

7. Procédé selon la revendication 6, dans lequel la présence ou l'absence des ARNs est déterminée en utilisant un colorant fluorescent ou une sonde marquée par fluorescence, éventuellement, dans lequel la sonde comprend en outre un fragment extincteur, éventuellement, dans lequel des ARNs sont amplifiés en utilisant une amorce RT tige-boucle.

8. Procédé selon l'une quelconque des revendications 1 à 5, dans lequel la présence ou l'absence d'ARNs est déterminée en utilisant une analyse d'hybridation.

9. Procédé selon l'une quelconque des revendications 1 à 5, dans lequel la présence ou l'absence des ARNs est déterminée par séquençage d'acides nucléiques, et des ARNs sont identifiés par un processus qui comprend l'élimination d'un adaptateur de séquençage 3' à partir de séquences d'ARNs individuelles.

10. Procédé selon l'une quelconque des revendications 1 à 9, dans lequel le sujet présente un allèle HTT sélectionné parmi un allèle à pénétrance totale, un allèle à pénétrance réduite, et un allèle à pénétrance intermédiaire.

11. Procédé selon l'une quelconque des revendications 1 à 10, dans lequel le sujet est asymptomatique.

12. Procédé selon l'une quelconque des revendications 1 à 11, dans lequel le sujet présente une MH de stade 1, une MH de stade 2, une MH de stade 3, ou une MH de stade 4.

13. Procédé selon l'une quelconque des revendications 1 à 12, dans lequel le procédé est répété, éventuellement dans lequel un sujet est évalué à une fréquence d'au moins environ une fois par an.

14. Procédé selon l'une quelconque des revendications 1 à 13, dans lequel le sujet suit une thérapie ou une thérapie candidate pour la MH ou des symptômes de MH.

15. Utilisation d'un kit d'évaluation d'échantillons axée sur l'activité de la maladie de Huntington, comprenant :
des sondes et/ou des amorces spécifiques de l'ARNs configurées pour réaliser le procédé selon l'une quelconque des revendications 1 à 14, pour détecter des ARNs selon SEQ ID NO : 6 ou SEQ ID NO : 1.

16. Utilisation selon la revendication 15, comprenant en outre des sondes et/ou des amorces spécifiques de l'ARNs pour détecter des ARNs selon SEQ ID NO : 7, SEQ ID NO : 10, SEQ ID NO : 2, SEQ ID NO : 3, SEQ ID NO : 9, SEQ ID NO : 11, SEQ ID NO : 12, SEQ ID NO : 22, SEQ ID NO : 23, SEQ ID NO : 25, SEQ ID NO : 26, SEQ ID NO : 27, SEQ ID NO : 55, SEQ ID NO : 40, SEQ ID NO : 41, SEQ ID NO : 137.
